# EUROPÄISCHE PATENTANMELDUNG

(11) **EP 2 664 330 A1**
(43) Veröffentlichungstag der Anmeldung: **20.11.2013**
(21) Anmeldenummer: 12168140.7
(22) Anmeldetag: 15.05.2012
(51) Int. Cl.: A61K 31/202, A61K 31/728, A61K 8/06, A61K 8/97, A61K 9/113, A61K 33/00, A61K 9/00, A61P 27/02

(54) **Zusammensetzung und Arzneimittel enthaltend Omega-3-Fettsäuren sowie einen Glucosaminoglucan**

(71) Anmelder: F. Holzer GmbH, 66386 St. Ingbert (DE)
(72) Erfinder: Steinfeld, Ute, 66386 St. Ingbert (DE); Holzer, Frank, 66386 St. Ingbert (DE); Lee, Hyeck Hee, 66386 St. Ingbert (DE); Mahler, Markus, 66333 Völklingen (DE)
(74) Vertreter: Pfenning, Meinig & Partner GbR

(57) **Zusammenfassung**

Die vorliegende Erfindung betrifft eine ophthalmologische Zusammensetzung, die mindestens eine ω-3-Fettsäure oder deren Derivat sowie mindestens ein Glucosaminoglucan enthält. Die ophthalmologische Zusammensetzung bewirkt bei Applikation am oder im Auge eine Proliferation des Augenepithels und somit ebenso eine Verbesserung der Wundheilung am Auge, insbesondere des Epithels. Damit wird eine effiziente Behandlung von strukturellen Schädigungen der Augenoberfläche gewährleistet. Weiterhin betrifft die Erfindung einen Fluidspender, der eine erfindungsgemäße ophthalmologische Zusammensetzung beinhaltet. Zudem betrifft die vorliegende Erfindung die Verwendung dieser Zusammensetzung für Kosmetische Zwecke.

## Beschreibung

Die vorliegende Erfindung betrifft eine ophthalmologische Zusammensetzung, die mindestens eine ω-3-Fettsäure oder deren Derivat sowie mindestens ein Glucosaminoglucan enthält. Die ophthalmologische Zusammensetzung bewirkt bei Applikation am oder im Auge eine Proliferation des Augenepithels und somit ebenso eine Verbesserung der Wundheilung am Auge, insbesondere des Epithels. Damit wird eine effiziente Behandlung von strukturellen Schädigungen der Augenoberfläche gewährleistet. Weiterhin betrifft die Erfindung einen Fluidspender, der eine erfindungsgemäße ophthalmologische Zusammensetzung beinhaltet. Zudem betrifft die vorliegende Erfindung die Verwendung dieser Zusammensetzung für Kosmetische Zwecke.

Sinnesorgane, wie Haut, Auge, Ohr, Nase und Mund und die angrenzenden Bereiche, wie etwa Hals-Rachenraum, Lunge und Bronchien, sind ständig mit der Außenwelt in Kontakt und damit Umwelteinflüssen ausgesetzt. Umwelt-Noxen, wie UV-Licht, Ozon, Stickstoffdioxid (NO2), Zigarettenrauch mit reaktiven Sauerstoffspezies und Aldehyden, die im Zigarettenrauch enthalten sind, sowie auch etwa trockene Heizungsluft, partikuläre Noxen, wie Schwebstäube, Viren, Bakterien, Pilzsporen und/oder Pollen können zu umweltinduzierten Störungen und Erkrankungen, wie z.B. Reizung der Schleimhäute, Augenbrennen, trockenem Husten, Abfall der Lungenfunktion, Kurzatmigkeit, Bronchitis, Lungenentzündungen, Asthma, Störungen des Immunsystems, insbesondere Allergien und Entzündungen, etc., führen.

Die Hornhaut (Cornea) ist der transparente, gewölbte vordere Abschnitt des Auges. Sie übt als äußerer Abschluss des Auges eine wichtige Schutzfunktion aus. Noch bedeutender ist ihre Funktion als Teil des optischen Systems. Durch ihre Krümmung wirkt sie wie eine Linse und spielt somit eine wichtige Rolle beim Sehvorgang. Eine klare, intakte und gut benetzte Cornea ist daher eine notwendige Voraussetzung für scharfes Sehen. Zum Schutz vor Verletzungen weist sie eine enge Versorgung mit reizempfindlichen Nerven auf, was sie gleichzeitig sehr schmerzempfindlich macht.

Das Epithel der Cornea besteht, von innen nach außen betrachtet, aus drei verschiedenen Zelltypen: den Basal-, Flügel- und Schuppenzellen.

Die Basalzellen besitzen die Eigenschaft sich unbegrenzt teilen zu können, wobei eine durch die Teilung entstandene Zelle als Flügelzelle in die nächst höhere Zelllage einwandert und die andere als Basalzelle in der untersten Schicht zurückbleibt. Die Flügelzellen bilden zwei bis drei Schichten im Epithel und werden bei ihrer Zellwanderung zur Außenseite flach. Nach weiterem Vordringen der Flügelzellen an die Oberfläche werden aus ihnen schließlich die Schuppenzellen. Diese sind wieder zwei- bis dreischichtig angeordnet. Eine abgestorbene Zelle wird dann durch die mechanischen Kräfte des Lidschlages, aus dem entsprechenden Zellverband gelöst, durch den Fluss des Tränenfilms zum Tränenpünktchen befördert und durch diesen letztlich abtransportiert.

Die Cornea ist einer ständigen Beanspruchung ausgesetzt, angefangen von natürlichen Faktoren über krankheitsbedingte, medizinischen Eingriffen geschuldeten bis hin zu künstlichen Faktoren wie z.Bsp.:
1) die Kraftwirkungen bei jedem Lidschlag durch die Augenlider
2) Fremdkörper aus der Atmosphäre (Staubpartikel, Pollen, Schmutz)
3) UV-Strahlung, Wind, geringe Luftfeuchtigkeit
4) falsche Tränenfilmzusammensetzung oder Tränenmangel
5) ausgetrocknete Muzine, besonders nach dem Aufstehen (= "Sandmännchen - Effekt")
6) Kontaktlinsen
7) chemische, physikalische und mechanische Einwirkungen (z.B. durch giftige Substanzen wie Putz- und Reinigungsmittel, Verätzungen, chirurgische Eingriffe, Lasik, mechanische Verletzungen)
8) Einnahme bestimmter Medikamente
9) Bestimmte Augenerkrankungen wie z.B. Blepharis, Sicca Syndrom, Keratitis, Allergien und Entzündungen. *Blepharis und Sicca Syndrom* sind zwei unterschiedliche Phänomene, die unabhängig voneinander auftreten, aber bei Patienten mit Augenproblemen aber auch oft gemeinsam beobachtet werden können. Sie können dauernde strukturelle Schäden verursachen und gelegentlich zum Verlust des Sehvermögens führen.
   Bei *Allergien und Entzündungen* wandern Immunzellen wie z.B. neutrophile Granulozyten in den Entzündungsherd ein, entlassen dort sie Proteasen (Kathepsin, Elastase u.a.) und bewirken eine Erhöhung von reaktiven Sauerstoffspezies, die zur Gewebeschädigung beitragen.
10) medizinische Eingriffe wie Hornhautchirugie, refraktive Chirugie (z.B. LASIK, PRK, LASEK,EPILASIK, FLEx, SmILE), Implantate, Transplantate.

Solche Faktoren können Reizungen und strukturelle Schäden in unterschiedlichen Ausmaß verursachen. Die möglichst schnelle Herstellung einer intakten Cornea ist sehr wichtig zur Erhaltung der Schutzfunktion, der unbeeinträchtigten Sicht und weil corneale Verletzungen sehr schmerzhaft sind.

Aus dem Stand der Technik sind ophthalmische Zusammensetzungen, beispielsweise in Form von Augentropfen oder Linsenaufbewahrungsflüssigkeiten, bekannt, die ω-3-Fettsäuren enthalten. Hierzu wird auf die WO 2006/007510 A1 sowie die WO 2007/130960 A2 verwiesen. Diese Zusammensetzungen liegen dabei als Öl-in-Wasser-Emulsion vor, wobei die dort beschriebenen Zusammensetzungen derart optimiert sind, dass diese Öl-in-Wasser-Emulsionen stabil sind. Diese Zusammensetzungen zeigen eine gewisse Wirksamkeit bei der Behandlung und Vorbeugung eines trockenen Augensyndroms (dry-eye-syndrom (DSE)).

Aufgabe der vorliegenden Erfindung ist es, ophthalmologische Präparate anzugeben, die eine Verbesserung der Wundheilung bei bestehenden Wunden am Auge erzielbar ist. Ebenso ist es Aufgabe der vorliegenden Erfindung, neuartige Kosmetika, insbesondere für den Gesichtsbereich bereitzustellen.

Diese Aufgabe wird bezüglich einer ophthalmologischen Zusammensetzung mit den Merkmalen des Patentanspruchs 1, bezüglich eines Fluidspenders mit den Merkmalen des Patentanspruchs 19 sowie bezüglich eines Kosmetikums mit den Merkmalen des Patentanspruchs 20 gelöst. Die jeweiligen abhängigen Patentansprüche stellen dabei vorteilhafte Weiterbildungen dar.

Erfindungsgemäß wird somit eine ophthalmologische Zusammensetzung, enthaltend
a) mindestens eine ω-3-Fettsäure und/oder ein ω-3-Fettsäurederivat ausgewählt aus der Gruppe bestehend aus Estern, Mono-, Di- oder Triglyceriden, Lipiden, Oxygenierungsprodukten, Carboxylatsalzen, Amiden, sonstigen pharmakologisch akzeptablen Carbonsäurederivaten und Mischungen hiervon, sowie
b) mindestens ein Glucosaminoglucan, hiervon abgeleitete pharmakologisch akzeptable Derivate, sowie Mischungen hiervon,
zur Prophylaxe und/oder Behandlung von strukturellen Epithelschäden, Schürfungen, Abrasionen, Verletzungen, Reizungen, Entzündungen, Verätzungen. Weiterhin Schäden, hervorgerufen durch Benetzungsstörungen aus der Gruppe, bestehend aus Sjögren-Syndrom, Sicca-Syndrom, Benetzungsstörungen des Auges bei Kontaktlinsenträgern, Lidrandentzündung (Blepharitis) und/oder chirurgischen Eingriffen am Auge, sowie zur Förderung oder Unterstützung der Wundheilung des Augenepithels und/oder zur Förderung der Proliferation der Epithelzellen des Auges.

Epitheliale Defekte nach operativen Eingriffen am Auge sind z.B. chirurgische

Eingriffe am vorderen Augenabschnitt wie Operation eines Kataraktes, eines Glaucoms, bei Hornhautchirugie, refraktiver Chirugie etc.

Die ophthalmologische Zusammensetzung stellt somit ein Kombinationspräparat aus w-3-Fettsäuren und Glucosaminoglucanen bzw. deren Derivaten dar. Die mindestens eine w-3-Fettsäure kann dabei als freie Säure enthalten sein, aber auch in derivatisierter, d.h. abgewandelter Form vorliegen. Als w-3-Fettsäurederivate im Sinne der vorliegenden Erfindung sind sämtliche Verbindungen zu verstehen, die sich von einer w-3-Fettsäure ableiten oder eine w-3-Fettsäure als strukturelles Element beinhalten. Darunter zählen somit bspw. Salze der w-3-Fettsäuren, Stoffe, die eine kovalent gebundene w-3-Fettsäure aufweisen oder Stoffgemische, die eine w-3-Fettsäure als integralen Bestandteil beinhalten.

ω-3-Fettsäuren sind mehrfach ungesättigte Fettsäuren und gehören zu den essentiellen Fettsäuren, die in der Regel mit der Nahrung aufgenommen und im Körper in Zellmembranen eingebaut werden.

Die Folgeprodukte dieser Fettsäuren sind Gewebshormone und gelten als körpereigene, regulatorisch wirksame Stoffe. Sie beeinflussen zahlreiche Stoffwechselvorgänge und -funktionen.

In Abhängigkeit spezieller Stimuli, z.B. durch neutrale Reize oder sonstige Mediatoren, z.B. Histamine, werden w-3- und w-6-Fettsäuren aus den Membranlipiden freigesetzt und für die Biosynthese von diesen Gewebshormonen, den Eicosanoiden, zur Verfügung gestellt.

Diese wirken bereits in sehr geringen Konzentrationen (zwischen 10⁻⁸ und 10⁻¹⁰ mol pro Liter) als Mediatoren unmittelbar am Ort ihres Entstehens. Die Effekte werden entweder auf parakrinem Wege auf benachbarte Zellen ausgeübt oder auf autokrinem Wege auf die produzierende Zelle selbst. Die Reichweite dieser Mediatoren wird durch ihre sehr kurze Lebensdauer von Sekunden bis wenigen Minuten begrenzt. So wird auch bei topischer Applikation oder Inhalation ein günstiger Einfluss auf die Zusammensetzung genommen und eine lokal begrenzte Wirkung erreicht, was vorteilhaft für die therapeutische Anwendung ist. Daneben nimmt die Fettsäurezusammensetzung auch Einfluss auf die Permeabilität und Fluidität der Membranen.

Unter Glucosaminoglucanderivaten sind Stoffe zu verstehen, die sich von einem Glucosaminoglucan ableiten oder ein Glucosaminoglucan als strukturelles Element beinhalten. Darunter zählen somit bspw. Salze der Glucosaminoglucane, Stoffe, die eine kovalent gebundene Glucosaminoglucan aufweisen oder Stoffgemische, die ein Glucosaminoglucan als integralen Bestandteil beinhalten.

Überraschenderweise wurde gefunden, dass mit der erfindungsgemäßen Zusammensetzung die Entstehung von Gewebereizungen und -schädigungen effizient vorgebeugt und vermieden werden kann, sowie bei bereits aufgetretenen Schädigungen eine beschleunigte Heilung hervorgerufen werden kann. Zudem ermöglichen die oben genannten ophthalmologische Zusammensetzungen eine gute Wundheilung bei Verletzungen des Auges, insbesondere des Augenepithels, z.B. Hornhaut und/oder Bindehaut des Auges. Die ophthalmologische Zusammensetzung bewirkt bei Applikation am oder im Auge eine Verbesserung der Wundheilung am Auge, insbesondere des Epithels und somit eine effiziente Behandlung von strukturellen Schädigungen der Augenoberfläche.

Nach Applikation der erfindungsgemäßen Zusammensetzung hat sich in Versuchen mit exzidierten Hornhäuten überraschend gezeigt, dass sich eine positive Veränderung der histologischen Struktur der Flügel und Deckzellen einstellen, die Proliferationsvorgänge bzw. eine strukturelle Veränderung der epithelialen Barriere belegen.

Die erfindungsgemäße Zusammensetzung erhält die Epithelien hervorragend. Auffallend ist, dass die erfindungsgemäßen Zusammensetzung eine positive Veränderung der histologischen Struktur der Flügel und Deckzellen bewirkt: im histologischen Schnitt erscheint das Epithel regelmäßig geschichtet, Flügelzellen obenauf und pallisadenförmige Basalzellen zeigen einen regelmäßigen Aufbau. Die vordere Stromalamelle ist sehr homogen verdichtet dargestellt.

In histologisch untersuchten Kulturen lässt sich eine Steigerung der Anzahl der Flügelzellschichten unter der Applikation mit der erfindungsgemäßen Zusammensetzung ableiten. Die Hornhaut entspricht sonst dem morphologischen Befund einer gesunden Hornhaut. Histologisch wurde in den untersuchten Kulturen, welche mit der erfindungsgemäßen Zusammensetzung betropft wurden, eine Anzahl von 4-5 Flügelzellschichten beobachtet. Üblicherweise sind in der Kaninchenhornhaut lediglich 2, maximal 3 Zellschichten histologisch nachweisbar. Die Zellproliferation innerhalb des Hornhautepithels wird beschleunigt, was eine verbesserte Wundheilung belegt.

Insbesondere fördert die erfindungsgemäße ophthalmologische Zusammensetzung die Wundheilung nach
- Chemischen, physikalischen und mechanischen Verletzungen am Auge, insbesondere der Hornhaut z.B. Verätzungen, Hornhautabschürfungen, Erosionen, operativen Eingriffen am Auge z.B. Lasik; chirurgische Eingriffe,
- Entzündungen im Bereich des Auges, z.B. Konjunktivitis (Entzündung der Bindehaut); Blepharitis (Entzündung der Augenlider); Keratitis - Entzündung der Hornhaut
- Zusammensetzung zur Behandlung und/oder Prävention von Epitheldefekten in der Hornhaut und/oder Bindehaut des Auges.

In einer bevorzugten Ausführungsform der Erfindung ist die mindestens eine ω-3-Fettsäure ausgewählt ist aus der Gruppe bestehend aus α-Linolensäure, Stearidonsäure, Eicosatetraensäure, Eicosapentaensäure (EPA), Docosapentaensäure (DPA), Docosahexaensäure, Hexadecatrienoensäure, Eicosatrienoensäure, Heneicosapentaenoensäure, Tetracosapentaenoensäure, Tetracosahexaenoensäure, hiervon abgeleitete Oxygenierungsprodukte, wie z.B. Alkohole, Ethylalkohole (wie z.B. Resolvin), Aldehyde, Ketone, Epoxide etc. sowie Mischungen oder Kombinationen hieraus. Die ω-3-Fettsäurederivate leiten sich somit bevorzugt von den zuvor genannten ω-3-Fettsäuren in entsprechender Weise ab.

Bevorzugte ω-3-Fettsäurederivate, d.h. Formen, in denen die mindestens eine ω-3-Fettsäure bevorzugt vorliegt sind dabei Ester eines organischen Alkohols, bevorzugt eines linearen oder verzweigten aliphatischen einwertigen Alkohols mit 1 bis 18 Kohlenstoffatomen, besonders Methyl-, Ethyl-, n-Propyl-, i-Propyl-, n-Butyl-, i-Butyl-, t-Butylester, wobei Ethylester ganz besonders zu bevorzugen sind.

Alternativ hierzu ist es ebenso bevorzugt, dass die mindestens eine ω-3-Fettsäure in Form eines pflanzlichen oder tierischen Öls enthalten ist, das neben der mindestens einen ω-3-Fettsäure noch mindestens eine ω-6-Fettsäure enthalten enthält. Das molare Verhältnis der ω-3-Fettsäure zur ω-6-Fettsäure beträgt hierbei vorteilhaft von 100:1 bis 1:100, bevorzugt 20:1 bis 1:10, weiter bevorzugt 15:1 bis 1:1, besonders bevorzugt von 8:1 bis 2:1. Bevorzugte Öle stellen dabei Algenöl, Fischöl, Perillaöl, Shiöl, Leinöl, Leindotteröl, Sacha Inchi Öl, Rapsöl, Olivenöl, Nachtkerzenöl, Sojaöl, Hanföl, Walnussöl, Erdnussöl, Sesamöl, Maiskeimöl Flachs-samenöl und/oder Mischungen hieraus dar.

Ebenso sind auch Kombinationsmöglichkeiten aus Estern und Ölen der mindestens einen ω-3-Fettsäure denkbar.

Beispiele für Omega-3 Fettsäure-Derivate sind die Resolvine (z.B. der Serie E und D, wie z.B. Resolvin E1 (RvE1) und Resolvin E2 (RvE2), sowie deren Isomere 18S-RvE1 und 18S-RvE2), Protectine (z.B. der Serie E und D, wie z.B.Protectin D1 und D2), Neuroprostane wie z.B. A4-NP, isoprostane etc. oder deren künstliche Analoga.

Der Gehalt der mindestens einen ω-3-Fettsäure und/oder des Derivats hiervon beträgt vorzugsweise, bezogen auf die gesamte Zusammensetzung, zwischen 0,01 und 60 Gew.-%, bevorzugt zwischen 0,05 und 30 Gew.-%, besonders bevorzugt zwischen 0,1 und 5Gew.-%

Gemäß einer weiter bevorzugten Ausführungsform ist das mindestens eine Glucosaminoglucan und/oder das hiervon abgeleitete Derivat ausgewählt aus der Gruppe bestehend aus Hyaluronsäure, Hyaluronaten, insbesondere Natriumhyaluronat, Chondroitinsulfat, Chondroitin, Keratinsulfat, Heparin, Heparinsulfat und/oder Mischungen hiervon.

Der Gehalt des mindestens einen Glucosaminoglucans und/oder des hiervon abgeleiteten Derivats beträgt vorzugsweise , bezogen auf die gesamte Zusammensetzung, von 0,001 bis 10 Gew.-%, bevorzugt von 0,005 Gew.-% bis 5 Gew.-%, besonders bevorzugt von 0,01 Gew.-% bis 1 Gew.-%.

Das mindestens eine Glucosaminoglucan und/oder das hiervon abgeleitete Derivat weist bevorzugt ein Molekulargewicht von 30.000 Dalton bis 10.000.000 Dalton, bevorzugt 250.000 bis etwa 5.000.000 Dalton auf.

Die Bestimmung des Molekulargewichts erfolgt dabei mit einem UBBELOHDE Viscometer (EP monograph method), dabei wird die intrinsische Viskosität der Polymere bestimmt und hieraus das gewichtsgemittelte Molekulargewicht errechnet.

Die ophthalmologische Zusammensetzung gemäß der vorliegenden Erfindung kann in Form einer Wasser-in-Öl-Emulsion, einer Öl-in-Wasser-Emulsion, einer Lösung, wässrigen Lösung, einer Suspension, einer Nanosuspensionen, einer Emulsion, einer Nanoemulsion, eines Gels, eines thixotropen Gels, eines Lipogels, eines Organogels, eines Mikroemulsionsgels, eines Hydrogels, eines Sprühgels, einer Paste, einer Creme, einer Salbe, eines in situ Gels oder eines Aerosols vorliegen. Ganz besonders bevorzugt sind dabei Wasser-in-Öl-Emulsion, Öl-in-Wasser-Emulsion, insbesondere jeweils als Mikroemulsion; oder Lösungen.

Mikroemulsionen sind dabei Emulsionen, bei denen der mittlere Teilchendurchmesser d₅₀ kleiner als 0,1 µm ist.

Für den Fall, dass die ophthalmologische Zusammensetzung gemäß der vorliegenden Erfindung als Öl-in-Wasser-Emulsion, insbesondere einer Öl-in-Wasser-Mikroemulsion, oder einer Wasser-in-Öl-Emulsion, insbesondere einer Wasser-in-ÖI-Mikroemulsion vorliegt, ist es insbesondere bevorzugt, dass einen Emulgator oder eine Kombination aus mehreren Emulgatoren, enthalten ist, wobei im Falle einer Öl-in-Wasser-Emulsion oder Öl-in-Wasser-Mikroemulsion der Emulgator oder jeder der Emulgatoren der Kombination bevorzugt einen HLB-Wert > 10 aufweist.

Der HLB-Wert einer Tensidmolekel in einer Grenzfläche wird durch die molekulare Verteilung und Orientierung der Molekel in den an dieser Grenzfläche beteiligten Phasen beschrieben. Für die Grenzfläche einer Tensidlösung gegen Luft wird das hydrophilehydrophobe Gleichgewicht durch Filmwaage- und Oberflächenspannungs-Messungen bestimmt.

Eine besondere Ausführungsform sieht vor, dass nur ein einziger Emulgator enthalten ist, dieser bevorzugt in einer Menge von < 1 Gew.-%, besonders bevorzugt ≤ 0,8 Gew.-%, insbesondere ≤ 0,6 Gew.-%, bezogen auf die gesamte Formulierung.

Bevorzugte Emulgatoren für die Öl-in-Wasser-Emulsion sind dabei ausgewählt aus der Gruppe bestehend aus ethoxylierten Castoröle und oder ethoxylierten hydrogenierten Castoröle, bevorzugt Magrogolglycerolricinoleat (z.B. Cremophor EL), Macrogolglycerolhydroxystearat (z.B. Cremophor RH40),, Glycerylsterate (z.B. Cutina 24 (PEG-20-Glyceryl stearat)) und/oder Macrogolcetylstearylether (z.B. CremophorA25) und in einer Menge von 0,01 bis 5 Gew.-%, bevorzugt von 0,05 bis 1 Gew-%, besonders bevorzugt von 0,1 bis 0,9 Gew.-%, insbesondere von 0,1 bis 0,8 Gew.%, bezogen auf die gesamte Zusammensetzung.

Bevorzugte Emulgatoren für die Wasser-in-Öl-Emulsion, insbesondere für die Wasser-in-Öl-Mikroemulsion sind ausgewählt aus der Gruppe der Lecithine, bevorzugt Epikuron 200, in einer Menge von 1 bis 10 Gew.-%, bevorzugt von 1 bis 7,5 Gew-% bezogen auf die gesamte Zusammensetzung

Zusätzlich zum einen Emulgator oder eine Kombination aus mehreren Emulgatoren kann mindestens ein Coemulgator enthalten ist, vorzugsweise PEG 400 und/oder 1,2-Propylenglykol enthalten sein.

Die erfindungsgemäße ophthalmologische Zusammensetzung ist bevorzugt konservierungsmittelfrei. Für den Fall, dass Konservierungsmittel enthalten sind, ist es besonders bevorzugt und ausreichend, dass lediglich Silberionen in der Zusammensetzung vorliegen. Die Silberionen können dabei in einem bevorzugten Konzentrationsbereich von 1 ppb bis 2 ppm, weiter bevorzugt von 10 ppb bis 1 ppm, enthalten sein. Die Anwesenheit der Silberionen kann durch den externen Zusatz von Silbersalzen, wie z.B. Silbernitrat zur Zusammensetzung resultieren, aber auch dadurch resultieren, dass die Zusammensetzung mit einem Gegenstand aus einer Silberlegierung oder massivem Silber kontaktiert wird, wodurch sich ein geringer Anteil des Silbers auflöst und in die Zusammensetzung übergeht. Etwaige Silber enthaltenden Gegenstände können z.B. Silberspiralen, wie sie in Dosiervorrichtungen für Flüssigkeiten oder Pasten vorkommen, sein. Entsprechende Dosiervorrichtungen ist z.B. aus der Patentanmeldung EP 1466 668 A1 bekannt. Die o.g. geringen Silberkonzentrationen sind ausreichend, um einen konservierenden Effekt, z.B. einen bakteriziden Effekt auf die Zusammensetzung auszuüben.

Insbesondere für den Fall, dass die Zusammensetzung Silberionen enthält, oder angedacht ist, die Zusammensetzung mit einem Silber haltigen Gegenstand in Kontakt zu bringen, ist es vorteilhaft, der Zusammensetzung mindestens einen Komplexbildner, ausgewählt aus der Gruppe der schwefelhaltigen organischen Verbindungen, insbesondere Natriumthiosalicylsäure, Thiosorbit, Cystein, N-Acetyl-L-cystein, Cyteinhydrochlorid, Cysteamin, Cystin, Methionin, Glutathion, S-Acetylglutathion, Thioglycerol, Thioharnstoff, Thiolactat; und/oder EDTA, EGTA sowie Kombinationen hieraus, bevorzugt in einer Menge von 0,0001 - 5 Gew.-%, vorzugsweise 0,0001-1 Gew.-%, bevorzugt 0,001-0,5 Gew.-%, bezogen auf die gesamte Zusammensetzung zuzusetzen. Die Komplexbildner komplexieren die Silberionen und verhindern dabei, dass die Silberionen von w-3-Fettsäuren komplexiert werden und somit die effektive Konzentration an w-3-Fettsäuren in der Zusammensetzung reduziert wird.

Alternativ hierzu kann die Zusammensetzung jedoch ebenso Konservierungsstoffe enthalten, z.B. mindestens ein in der Ophthalmologie gebräuchliches Konservierungsmittel in einer Menge von 0,001 - 1 Gew.-%, vorzugsweise 0,01-0,5 Gew.-%, bevorzugt 0,01-0,04 Gew.-%, bezogen auf die gesamte Zusammensetzung, vorzugsweise ein Konservierungsmittel ausgewählt aus der Gruppe bestehend aus Polyquad, Natriumperborat, Purite; Alkohole, wie Chlorobutanol, Benzylalkohol, Phenoxyethanol; Carboxylsäuren wie Sorbinsäure; Phenole, wie Methly-/ Ethylparaben; Amidine wie Chorhexidindigluconat; quaternäre Ammoniumverbindungen wie Benzalkoniumchlorid, Benzethoniumchlorid und Cetylpyridiniumchlorid, Benzylbromid und/oder Kombinationen hieraus.

In einer weiteren bevorzugten Ausführungsform ist die ophthalmologische Zusammensetzung phosphatfrei und/oder Calciumionenfrei, d.h. sie enthält jeweils weniger als 0,3 mmol/l Phosphat- und/oder Calciumionen, vorzugsweise jeweils weniger als 0,1mmol/l Phosphat- und/oder Calciumionen und besonders bevorzugt keine Phosphat- und/oder Calciumionen.

Weiter bevorzugt kann die ophthalmologische Zusammensetzung einen Chelatbildner für Calciumionen enthalten, bevorzugt aus der Gruppe der Citratsalze, Citronensäure, EDTA, EGTA und Mischungen davon enthält, bevorzugt in einer Konzentration von 0,05-10 Gew-%, bevorzugt 0,1 Gew-%, besonders bevorzugt 0,5-3 Gew-% bezogen auf das Gesamtgewicht der pharmazeutischen Zusammensetzung.

Die ophthalmologische Zusammensetzung der vorliegenden Erfindung kann weitere Bestandteile umfassen oder beinhalten; eine Liste beispielhafter weiterer Komponenten ist nachfolgend aufgeführt:
a) pharmakologisch geeignete Trägerstoffe, insbesondere Wasser, Fettsäureester, wie Isopropylpalmitat und/oder Isopropylmyristat; Shingolipide wie etwa Ceramide, Cerobroside, Phosphatidylethanolamine, Glycerol, Neutralöle wie Miglyol, Ethyloleat, Capryl-Caprinsäure-Triglycerid, Miglyol, Wachse; Fette; Vaseline; Paraffine; Mineralöl und/oder Pflanzenöl, bevorzugt in einer Menge zwischen 50 und 95 Gew.-%, bevorzugt zwischen 75 und 90 Gew.-%, bezogen auf die gesamte Zusammensetzung,
b) mindestens einen pflanzlichen anti-inflammatorischen und/oder antioxidativen Stoff, ausgewählt aus der Gruppe bestehend aus Flavonoiden (z.B. Rutin, Quercetin, Curcurmin), Isoflavonoiden (z.B. Silymarin), Polyphenole (z.B. Resveratol), Anthocyanen, Triterpenen, Monoterpenalkoholen, Phenolcarbonsäuren, Carotinoiden (z.B. β-Carotin, α-Carotin, Lycopin, β-Cryptoxanthin, Lutein, Zeaxanthin), Retinoide (z.B. Tretinoin), Tocopherolen (Vitamin E) und Biotin, Vitamine A, C, D, K, Coenzym Q (=Q10), Carnitin, N-Acetyl-Carnitin, Carnesol, Ubichinon und/oder Taurin, bevorzugt in einer Menge zwischen 0,01 und 5 Gew.-%, bezogen auf die gesamte Zusammensetzung,
c) mindestens einen pflanzliche Stoff mit antioxidativer, antiinflammatorischer und/oder antiallergischer Wirkung, z.B. pflanzliche Einzelstoffe, Stoffgemische, ein flüssiger oder fester Extrakt, ein Destillat oder ein Öl oder ätherisches Öl, bevorzugt aus Pflanzen der Gattungen oder Arten Rosmarin, Sanddorn, Myrrhe, Eupharis (Augentrost), Kamille, Arnika, Ringelblume, Thymian, Echina, Calendula, Teebaum, Teestrauch, Apfelbeere (Aronia), Ginkgo, Gin-seng, Heidelbeere, Holunder, Lavendel, Anis,
d) mindestens einen Wirkstoff, ausgewählt aus der Gruppe bestehend aus Antibiotika, abschwellenden Medikamenten, nichtsteroidalen Antiphlogistika, Virustatica, Antiseptika, Kortison, antiallergischen Wirkstoffen, Prostaglandin-Analoga, Wirkstoffen aus der Wirkstoffklasse der Antihistaminika und/oder der Corticosteroide, antiallergischen Wirkstoffe, Pantothensäurederivaten, nichtsteroidalen Entzündungshemmer, Vaskokonstriktoren und/oder Anti-Glaucom-Wirkstoffen in einer pharmakologisch Wirksamen Konzentration,
e) mindestens einen Gelbildner, ausgewählt aus der Gruppe bestehend aus natürlichen oder synthetischen Polymeren, bevorzugt in einer Menge zwischen 0,01 und 5 Gew.-%, bezogen auf die gesamte Zusammensetzung.
f) mindestens ein Verdickungsmittel, bevorzugt in einer Menge zwischen 0,5 und 5 Gew.-%, bezogen auf die gesamte Zusammensetzung.
g) mindestens ein Feuchthaltemittel,
h) mindestens eine Hilfsstoff, ausgewählt aus der Gruppe bestehend aus anorganischen Puffersubstanzen, organischen Puffersubstanzen, anorganischen Salzen, organischen Salzen, Viskositätsreglern, Lösungsmitteln, Lösungsvermittlern, Lösungsbeschleunigern, Salzbildnern, Viskositäts- und Konsistenzbeeinflussern, Gelbildnern, Solubilisatoren, Benetzern, Spreizmitteln, Füll- und Trägerstoffen, Osmolaritätsreglern so-wie Mischungen davon, sowie
i) Kombinationen aus den zuvor genannten Bestandteilen.

Konservierungsmittel sind antimikrobielle Biozide zur Ausschaltung von Mikroorganismen.

Antioxidantien sind Verbindungen, die eine unerwünschte Oxidation anderer Substanzen gezielt verhindert.

Hierbei umfasst Vitamin A das Vitamin A1 (Retinol), Vitamin A2 (3-Dehydroretinol), Vitamin-A-Säure, Vitamin-A-Abkömmlinge (Retinylpalmitat, Retinylacetat etc.), all-trans-Retinolsäure (ATRA, aRA, Tretinoin), 13-cis-Retinolsäure bzw. -Retinsäure (Isotretinoin), Vitamin A-Analoge wie z.B. die all-trans Retinsäure.

Vitamin C umfasst Ascorbinsäure, Ascorbylpalmitat und Ascorbylacetat und Vitamin E umfasst Gamma-Tocotrienol und 6-hydroxy-2,5,7,8-tetramethyl-chroman-2-carboxylsäure (Trolox).

Beispiele für Antioxidantien sind Terpenoide (Monoterpenoid, Sesquiterpenoid, Diterpenoid, Triterpenoid), Carotinoide (α- und β-Carotin), Hydroxy-tyrosol, Zeathin, Lutein, Lycopene, Anthocyanine, Cryptoxan-thine, Xanthophylle, Epicatechin, Quercetin, Punicalagine und Ellagsäure; Chlorogensäure, Gallensäure, Ferulasäure, Kaffeesäure, α-Tocopherol, α-Tocopherolester, Ascorbinsäure, Ascorbinsäureester ( myristat, -palmitat und -stearat), β-Carotin, Cystein, Acetylcystein (N-Acetyl-L-cystein stellt auch gleichzeitig ein mucolytisches Mittel dar), Coenzym Q, Idebenone (synthetisches Quinone ähnlich Q10), Folsäure (Vitamin-B2-Gruppe), Phytinsäure, cis-und/oder trans-Uro-cansäure, Karnosin (N-β-Alanin-L-Histidin), Histidin, Flavone, Flavonoide, Lycopin, Taurin, Tyrosin, Gluthation, Gluthationester, α-Liponsäure, Ubichinon, Niacin, Nordihydroguaiaretsäure, Gallussäureester (Ethyl-, Propyl-, Octyl-, Dodecylgallat), Phosphorsäurederivate (Monophosphate, Polyphosphate), Butylhydroxytoluol, Butylhydroxyanisol, Tetraoxydimethylbiphenyl, Tocotrienole (Teil der Vitamin-E-Stoffgruppe), Polyalkohole, Polyphenole, Zitronensäure, Weinsäure, Edetinsäure (EDTA als DiNa- oder DiNaCa-Salz), Coniferylbenzoat und/oder deren Derivate als Antioxidationsmittel.

Die Antioxidantien können direkt oder auch in Form von Ölen oder ätherischen Ölen zugesetzt werden.

Weizenkeimöl enthält z.B. Tocopherole, Karotinoide, Ergocalciferol, Folsäure (Vitamin B9), Panthoten-säure, Phytosterine und Phenole, wie Dihydroquercetin, etc.

Für die erfindungsgemäßen Zusammensetzungen geeignete Gelbildner umfassen vorzugsweise natürliche oder synthetische Polymere. Natürliche Polymere sind vorzugsweise ausgewählt aus der Gruppe bestehend aus Agar-Agar, Alginsäure, Alginat, amidiertes Pektin, Propylenglycolalginat, Carbomer, Carrageenan, Casein, Cellulose-Derivate (Methyl-, Hydroxyethyl, Carboxymethylcellulose-Natrium), Dammar-Gummi, Dextrine, Furcellaran, Gelatine, Guargummi, Guarkernmehl, Gellan, Ghatti-Gummi, Gummi arabicum, Gummi aus Fichtensaft, Johannisbrotkernmehl, Karaya-Gummi, Keratin, Konjakmehl, L-HPC, Locust Bean Gum, Mastix, Pektin, Schellack, (ggf. modifizierte) Stärke, Tarakernmehl, Traganth, Xanthangummi und deren Derivate. Bevorzugte synthetische Polymere, die als Geliermittel für die erfindungsgemäße Zusammensetzung eingesetzt werden können, sind ausgewählt aus der Gruppe bestehend aus Acrylsäurepolymeren, Carbomeren, Polyacrylamiden und Alkylenoxidpolymeren.

Verdickungsmittel, die vorzugsweise in den erfindungsgemäßen Zusammensetzungen enthalten sein können, umfassen beispielsweise Candelilla und Carnaubawachs sowie mikrokristalline Wachse, Carbomer, Polyethylenoxid-Verdicker, Polaxamere, Hydroxyethylcellulose, Hydroxypropylcellulose, Hypromellose, Povidon, Hyaluronsäure, Polymilchsäure und Derivate davon. Das Verdickungsmittel wird vorzugsweise in einer Menge von 0,5 bis 5 Gew.-%, bezogen auf das Gesamtgewicht der erfindungsgemäßen, pharmazeutischen Zusammensetzung, eingesetzt.

Beispiele für pflanzliche Extrakte sind hierbei:
- pflanzliche Stoffe mit antioxidativer, anti-in-flammatorischer und oder antiallergischer Wirkung, z.B. pflanzliche Einzelstoffe, Stoffgemische, ein flüssiger oder fester Extrakt, ein Destillat oder ein Öl oder ätherisches Öl, der/das aus Pflanzen, z.B. der nachfolgend genannten Gattungen oder Arten, ist:
- Borretsch, Eupharis (Augentrost), Nachtkerze, Hamamelis, Sonnenhutkraut, Kamille, Arnika, Ringelblume, Thymian, Aloe vera, Salbei, Minze, Pfefferminz, Johanniskraut, Rosmarin, Sanddorn, Cardiospermum halicacabum, Myrrhe, Ratanhia, Fenchel, Weide, Schafgarbe, Huflattich, Beinwell, Teufelskralle, Bittersüß, Holunder, Eukalyptus, Echina, Calendula, Teebaum, Teestrauch, Süßholz, Ma-tisse, Koriander, Tausendgüldenkraut, Brennnessel, Ananas, Fichte, Kiefer, Tanne, Eiche, Apfelbeere (Aronia), Ginkgo, Ginseng, Heidelbeere, Holunder, Lavendel, Anis, Grape-fruit, Zitrone, Wintergras, Ananas, Isländisch Moos;
- pflanzliche anti-inflammatorische und/oder antioxidative Stoffe aus der Gruppe bestehend aus Gerbstoffen, ätherischen Öle, Azulenen, Proazulenen, Bisabololen, Bisaboloiden, Flavonoiden (z.B. Rutin, Quercetin), Flavonen, Anthocyanen, Triterpenen, Monoterpenalkoholen, Phenolcarbonsäuren, Polyphenolen, ungesättigten Fettsäuren, Hypericin, Carotinoiden, Allantoin, Bromelain, Glycyrrhizin, Glycyrrhizinsäure und Salze der Glycyrrhizinsäure;
- anti-inflammatorische Wirkstoffe ausgewählt aus der Gruppe bestehend aus Vitamin A, Carotinen, Carotinoiden, z.B. β-Carotin, α-Carotin, Lycopin, β-Cryptoxanthin, Lutein, Zeaxanthin, Tretinoin, Tocopherolen (Vitamin E) und Biotin, Vitamine A, C, D, K, Q10, Pangamsäure, Honig, Gelee Royal, Casein;
- pflanzliche Öle mit anti-inflammatorischer Wirkung, vorzugsweise ausgewählt aus der Gruppe bestehend aus Perilla-Öl, Chi-Öl, Fischöl, Algenöl, Nachtkerzenöl, Borretschöl, Weizenkeimöl, Rapsöl, Sojaöl; sowie
- anti-inflammatorische, antioxidative Stoffe ausgewählt aus der Gruppe bestehend aus pflanzlichen Gerbstoffen und synthetischen Gerbstoffen.

In einer weiteren bevorzugten Ausführungsform enthält die Zusammensetzung mindestens ein Feuchthaltemittel, wie z.B. Glycerin, Glykole, Sorbitol. Diese erhöhen nicht nur die Feuchtigkeit im Gewebe, sondern haben auch einen biostatischen Effekt.

Insbesondere ist es vorteilhaft, wenn die Zusammensetzung mindestens einen weiteren Wirkstoff enthält, der natürliche, synthetische oder biotechnologisch hergestellt sein kann. Dieser zusätzliche Wirkstoff kann dabei ausgewählt sein aus der Gruppe bestehend aus Antibiotika, abschwellenden Medikamenten, nichtsteroidalen Antiphlogistika, Virustatica, Antiseptika, Kortison, antiallergischen Wirkstoffen, Prostaglandin-Analoga, Wirkstoffen aus der Wirkstoffklasse der Antihistaminika und/oder der Corticosteroide, antiallergischen Wirkstoffe, Pantothensäurederivate, nicht-steroidalen Entzündungshemmer, Vasokonstriktoren und/oder Anti-Glaucom-Wirkstoffen, FP-Prostanoid-Rezeptorantagonisten, Prostamidrezeptor-Antagonisten und/oder natürliche oder synthetische Inhibitoren oder Antagonisten von TNF alpha.

Die Wirkstoffe können dabei ausgewählt sein aus natürlichen, synthetischen oder biotechnologisch hergestellten Wirkstoffen. Spezielle Beispiele hierfür werden nachfolgend angegeben:
Antibiotika:
   - Polypeptid-Antibiotika: Bacitracin, Polymyxin B, Gramicidin,
   - Aminoglykoside: Neomycin, Framycetin, Gentami-cin, Tobramycin,
   - Sulfonamide: Sulfacetamid,
   - Chinolone: Ciprofloxacin, Ofloxacin, Lomefloxacin, Moxifloxacin,
   - andere Antibiotika: Chloramphenicol Fusidinsäure
Alternativ in Kombination mit okulären Glucocorticoiden
   - abschwellende Medikamente, wie Naphazolin, Phenylephrin, Tetryzolin, Tramazolin Xylometazolin;
   - nichtsteroidale Antiphlogistika, wie Diclofenac, Indometacin;
   - Virustatica, wie Aciclovir;
   - Antiseptika, wie Kortison, wie Hydrocortison, Rimexolon;
   - antiallergische Wirkstoffe aus der Antihistaminika, Corticosteroide, synthetische Mastzelldegranulationshemmer und Leukotrien-Rezeptor-Antagonisten;
   - Prostaglantin-Analoga, Antibiotika;
   - mindestens ein Wirkstoff aus der Wirkstoffklasse der Antihistaminika und/oder mindestens ein Wirkstoff aus der Wirkstoffklasse der Corticosteroide;
   - die Gruppe der Antihistaminika Ketotifen, Thonzylamin, Mepyramin, Thenalidin, Tripelenamin, Chlorpyramin, Promethazin, Tolpropamin, Dimetinden, Clemastin, Bamipin, Isothipendyl, Diphen-hydramin, Diphenhydraminmethylbromid, Chlorphe-noxamin, Pheniramin, Diphenylpyralin, Dioxopro-methazin, Dimenhydrinat, Thiethylperazin und Meclozin, Azelastin, Levocabastin, Astemizol, Mebhydrolin, Terfenadin, Mequitazin, Cetirizin, Emedastin, Mizolastin, Olopatadin, Epinastin und Antazolin;
   - die Gruppe der Corticosteroide Triamcinolon, Dexamethason, Hydrocortison, Hydrocortison-acetat, Hydrocortisonbutyrat, Hydrocortisonbuteprat, Prednisolon, Betamethason, Methyl-prednisolon, Clobetason, Flumetason, Fluocortin, Fluperolon, Fluorometholon, Flupredniden, Desonid, Triamcinolon, Alclometason, Dexamethason, Clocortolon, Betamethason, Fluclorolon, Desoxi-metason, Fluocinolonacetonid, Fluocortolon, Diflucortolon, Fludroxycortid, Fluocinonid, Budesonid, Diflorason, Amcinonid, Halometason, Mometason, Methylprednisolonaceponat, Beclo-metason, Hydrocortisonaceponat, Fluticason, Prednicarbat, Prednison, Prednisolon, Diflu-prednat, Ulobetasol, Clobetasol, Halcinonid, Medryson, Desonid, Formocortal, Rimexolon, Mazipredon, Flunisolid und Tixocortol;
   - mindestens ein antiallergischer Wirkstoff aus der Gruppe Cromoglicinsäure, Spagluminsäure, Lodoxamid, Nedocromil, Montelukast und Zafirlukast;
   - Pantothensäurederivate Dexpanthenol, DL-Panthenol, Salze der Pantothensäure (z.B. Na-Pantothenat, Ca-Pantothenat), Ester der Pantothensäure (z.B. Ethyl-, Methylester), Panthenol-Ether (z.B. Ethyl- oder Methylether), Panthenol-Thioether sowie Panthenyltriacetat, besonders bevorzugt Dexpanthenol (= D-(+)-Pantothenyl-alkohol);
   - alternativ nicht-steroidale Entzündungshemmer ("NSAIDs"), wie z.B. Aminoarylcarbonsäure-Derivate (z.B. Enfenaminsäure, Etofenamat, Flufenaminsäure, Isonixin, Meclofenaminsäure, Mefen-aminsäure, Nifluminsäure, Talniflumat, Tero-fenamat, Tolfenaminsäure), Arylacetalsäure-Derivate (z.B. Aceclofenac, Acemetacin, Alclofenac, Amfenac, Amtolmetinguacil, Bromfenac, Bufexamac, Cinmetacin, Clopirac, Diclofenacnatrium, Etodolac, Felbinac, Fenclozinsäure, Fentiazac, Glucametacin, Ibufenac, Indomethacin, Isofezolac, Isoxepac, lonazolac, Metiazinsäure, Mofezolac, Oxametacin, Pirazolac, Proglumetacin, Sulindac, Tiaramide, Tolmetin, Tropesin, Zomepirac), Aryl-Buttersäure-Derivate (z.B. Bumadizon, Butibufen, Fenbufen, Xenbucin), Arylcarbonsäure (z.B. Clidanac, Ketorolac, Tinoridin), Arylpropionsäure-Derivate (z.B. Alminoprofen, Benoxaprofen, Bermoprofen, Bucloxsäure, Carprofen, Fenoprofen, Flunoxaprofen, Flurbiprofen, Ibuprofen, Ibuproxam, Indoprofen, Ketoprofen, Loxoprofen, Naproxen, Oxaprozin, Piketoprolen, Pirofen, Pranoprofen, Protizinsäure, Suprofen, Tiaprofensäure, Ximoprofen, Zaltoprofen), Pyrazole (z.B. Difenamizol, Epirizol), Pyrazolone (z.B. Apazone, Benzpiperylone, Feprazone, Mofebutazone, Morazone, Oxyphenbutazone, Phenylbutazone, Pipebuzone, Propyphenazone, Ramifenazone, Suxibuzone, Thiazolinobutazone), Salicylsäure-Derivate (z.B. Acetaminosalol, Aspirin, Benorylate, Bromosaligenin, Calciumacetylsalicylate, Diflunisal, Etersalate, Fendosal, Gentisinsäure, Glycoisali-cylate, Imidazolsalicylate, Lysinacetylsalicylate, Mesalamine, Morpholinsalicylate, 1-Naphthylsalicylate, Olsalazine, Parsalmide, Phenyl-acetylsalicylate, Phenylsalicylate, Salacetamide, Salicylamide o Essigsäure, Salicylsulfonsäure, Salsalate, Sulfasalazine), Thiazincarboxamide (z.B. Ampiroxicam, Droxicam, Isoxicam, Lornoxicam, Piroxicam, Tenoxicam), ε-Acetamidcapronsäure, S (5`-adenosyl)-L-methionine, 3-amino-4-hydroxy-Buttersäure, Amixetrine, Bendazac, Benzydamine, α-Bisabolol, Bucolome, Difenpiramide, Ditazol, Emorfazon, Fepradinol, Guaiazulene, Nabumetone, Nimesulide, Oxaceprol, Paranyline, Perisoxal, Proquazone, Superoxid-Dismutase, Tenidap, Zileulon, deren physiologisch akzeptablen Salze sowie Kombinationen und Mischungen hiervon.

Andere nicht-steroidale Entzündungshemmer ("NSAIDs"), die zusätzlich in der erfindungsmäßigen Zusammensetzung enthalten sein können, umfassen Cyclooxygenase-Inhibitoren, z.B. selektive Inhibitoren der Cyclooxy-genase vom Typ II, wie z.B. Celecoxib und Etodolac, PAF (platelet activating factor)-Antagonisten, wie etwa Apafant, Bepafant, Minopafant, Nupafant, und Modipafant; PDE (Phosphodiesterase)-IV-Inhibitoren, wie etwa Ariflo, Torbafylline, Rolipram, Filaminast Piclamilast, Cipamfylline und Roflumilast; Inhibitoren der Cytokine-Bildung, wie etwa Inhibitoren des NF-κB-Transkriptionsfaktors; oder andere bekannte anti-entzündliche Agenzien.

Die erfindungsgemäße pharmazeutische Zusammensetzung kann aus der Gruppe der Vasokonstriktoren z.B. Oxy-metazolin, Xylometazolin, Tretryzolin, Naphazolin, Tramazolin und/oder deren Derivate als Wirkstoffkomponente enthalten.

Zusätzlich zu den Fettsäuren können gegebenenfalls auch noch Anti-Glaucom-Wirkstoffe, wie
- Beta-Blocker: Timolol, Levobunolol,
- Cholinergika: Carbachol, Pilocarpin,
- Alpha-2-Adrenorezeptor-Agonist: Clonidin, Brimonidin, Carboanhydrasehemmer: Brinzolamid, Dor-zolamid und Acetazolamid,
- Prostaglandine: Latanoprost, Travoprost, Bimatoprost, Tafluprost,
zugesetzt werden, um noch stärker auf die Wirkung Einfluss zu nehmen.

Die Konzentration der alternativ zugesetzten Agenzien, die in der vorliegenden Erfindung enthalten sind, kann je nach Agenz und Typ der Erkrankung variieren. Die Konzentration soll ausreichen, um z.B. eine Entzündung im behandelten Gewebe zu behandeln oder dieser vorzubeugen. Typischerweise liegen die Konzentrationen dabei im Bereich von 0,0001 bis etwa 5 % wt/wt (oder alternativ bei 0,01 bis etwa 2 % wt/wt, oder von etwa 0,05 % bis 1 % wt/wt, oder von etwa 0,01 % bis etwa 0,5 % wt/wt).

Ebenso ist es möglich, dass als Wirkstoffe Modulatoren der COX-2 enthalten sind. Beispiele für natürliche Inhibitoren sind Basilikum, Berberine, Curcumin, EGCG, Ingwer, Hopfen (Humulus lupulus), Fischöl, Oregano, Quercetin, Resveratrol, Rosemarie sowie die Vitamine A und E.

Ebenso kann können zusätzlich Modulatoren, z.B. Inhibitoren, Antagonisten, insbesondere Modulatoren des NF-κB-Transkriptionsfaktors enthalten sein.

Immunologische Prozesse, Prozesse in Entzündungsverläufen und Wundheilungsprozesse bilden ein eng verwobenes Zusammenspiel bei Reizungen, Entzündungen und Heilungsvorgängen und sind untrennbar miteinander verknüpft. Modulatoren sind Stoffe, die in dieses komplexe Zusammenspiel regulierend/modulierend einwirken und so die optimale Funktion des Immunsystems unterstützen und/oder einen positiven Effekt auf die Prophylaxe oder Behandlung von Reizungen, Entzündungen und/oder die Wundheilung ausüben.

Folgende Modulatoren physiologischer Prozesse, wie Reizungen, Entzündungsverläufe und/oder Wundheilung, sind aus dem Stand der Technik bekannt und vom oben verwendeten Begriff Modulator umfasst:
- Coenzym Q10 (Q10, Ubichinon-10). Q10 ist essentiell auf mitochondrialer Ebene zur optimalen Funktion des Immunsystems (Folkers K, Wolaniuk A; Research on coenzyme Q10 in clinical medicine and in immunomodulation. Drugs Exp Clin Res. 1985;11(8):539-45). Q10 wirkt in entzündlichen Prozessen auf der Ebene der Genexpression. Es übt u.a. anti-inflammatorische Effekt über Einfluss auf NFkappaB1-abhängige Gen-Expression aus (Schmelzer C, Lindner I, Rimbach G, Niklowitz P, Menke T, Döring F., Functions of coenzyme Q10 in inflammation and gene expression, Biofactors 2008;32(1-4):179-83). Q10 kann in einer Konzentration von 1-100 µm, bevorzugt in einer Konzentration von 2-10 µM, verwendet werden;
- Taurin. Taurin kommt in Immunzellen vor und moduliert bestimmte Immunzeilfunktionen, so z.B. Regulation von inflammatorischen Aspekten der Immunantwort. Es wirkt auch als Schutz in seiner Funktion als Antioxidants (Fazzino F, Obregón F, Lima L. Taurine and proliferation of lymphocytes in physically restrained rats. J Biomed Sci. 2010 Aug 24;17 Suppl 1:S24) und als Osmoregulator (Shioda R., Reinach P.S., Hisatsune T., Miyamoto Y. Osmosensitive taurine transporter expression and activity in human corneal epithelial cell. IOVS, Sept. 2002, Vol. 43, No. 9). Taurin kann in einer Konzentration von 0,1-50 mM, bevorzugt 0,1-5 mM, verwendet werden;
- Carboxymethylcellulose (CMC). CMC bindet an humane Epithelzellen und ist ein Modulator der kornealen epithelialen Wundheilung (Invest Ophthalmol Vis Sci. 2007 Apr;48(4):1559-67). CMC-Bindung an die Matrix stimuliert die Anheftung, Migration und Reepithelialisierung kornealer Wunden in HCEC;
- Resolvin (insbesondere die E- und D-Serie). Resolvin E1 (RvE1) induziert eine Zunahme der Zell-Migration und damit eine beschleunigte epitheliale Wundheilung (Zhang et al, IOVS, Vol. 51, No. 11, November 2010); und
- Protectine. Protectine sind, wie Resolvine, Derivate der Eicosapentaensäure und Docosahexaensäure üben anti-inflammatorische Effekte aus (Curr Opin Clin Nutr Metab Care. 2011 Mar;14(2):132-7.Docosahexaenoic acid, protectins and dry eye. Cortina MS, Bazan HE)

Überraschenderweise wurde gefunden, dass mit der erfindungsgemäßen Zusammensetzung zum einen die Entstehung von Gewebereizungen und - schädigungen von Allergien oder Entzündungen effizient vorgebeugt und vermieden werden kann, sowie bei bereits aufgetretenen Schädigungen in das Entzündungsgeschehen eingegriffen werden kann, wobei sich eine weitere Verbesserung der Wundheilung erzielen lässt.

Wenn Gewebereizungen oder -schädigungen erfolgen, sei es durch Umweltnoxen, mechanische Reize, wie Reibung, Druck, durch Bakterien, Trauma, Chemikalien, Hitze und/oder überschießende Immunreaktionen, wie Allergien, etc., kommt es zunächst zu Aktivitätsänderungen in bestimmten zellulären Signalwegen, die wiederum zu spezifischen Änderungen des Genexpressionsmusters führen.

Es werden in den betroffenen Geweben diverse Entzündungsmediatoren freigesetzt, die Entzündungsprozesse einleiten und aufrechterhalten. Die Gesamtheit dieser komplexen Gewebeveränderungen wird als Entzündung bezeichnet. Reguliert ablaufende Entzündungen spielen bei den Prozessen der Wundheilung eine wichtige Rolle.

Die bevorzugte, modulatorhaltige Zusammensetzung greift zur Vermeidung, Modulation oder Hemmung auf verschiedenen Ebenen in diese komplexen Vorgänge ein:
Durch die erfindungsgemäße Zusammensetzung in Kombination mit einem Modulator, z.B. einem Inhibitor, Antagonist, etc., des NF-κB-Transkriptionsfaktors einsetzt, wird die Entstehung entzündungshemmender Mediatoren begünstigt, um prophylaktisch oder therapeutisch Entzündungen vorzubeugen, so dass eine weitere Verbesserung der Abheilung bestehender Verletzungen des Auges begünstigt wird, da Entzündungen, die die Abheilung negativ beeinflussen, unterdrückt werden können. Ebenso wurde jedoch auch die Abheilung bei bereits bestehenden Entzündungen beobachtet.

Einer der wichtigsten intrazellulären Regulatoren von Entzündungsreaktionen ist der Transkriptionsfaktor NF-κB, der durch verschiedene Formen von Zellstress aktiviert wird, etwa chemisch-physikalische Noxen, bakterielle und virale Antigene, Zytokine, etc., und die Genexpression in betroffenen Zellen schnell und umfassend ändern kann. Unter den Genen, die entsprechend hoch reguliert werden, befinden sich insbesondere Zytokine, wie IL-1, TNFalpha, Enzyme, wie COX-2, iNOS, Zelladhäsionsmoleküle, etc., die für eine Ausbreitung der Entzündungsreaktion auf andere Zellen und deren Verstärkung, oft im Sinne einer positiven Rückkopplung, sorgen.

Die Modulation der Aktivierung von NF-κB stellt eine weitere Möglichkeit dar, mit der die erfinderische Zusammensetzung in das Entzündungsgeschehen eingreifen kann.

Modulatoren des NF-kappa B Transkriptionsfaktors können z.B. direkt auf NF-kappa B wirken, oder indirekt über die Signalkaskade auf NF-kappa B. Antioxidantien z.B. können Komponenten des NF-kappa B Signaltransduktionsweges inhibieren, einschließlich des TNF Rezeptors und des Proteasoms.

Durch die Modulation von NF-kappa B kann sich eine Modulation (insbesondere Suppression) des Tumor-Nekrose-Faktors alpha (TNF-α) ergeben. TNF-α ist ein Signalstoff des Immunsystemes und spielt bei Erkrankungen wie z.B. beim Sjögren-Syndrom, der Keratoconjunctivitis sicca, Erkrankungen der Meimom-Drüse eine große Rolle.

Während des Entzündungsprozesses werden große Mengen an reaktiven Sauerstoff- und Stickstoffspezies gebildet, die u.a. auch regulatorisch in den Entzündungsprozess eingreifen. So wird etwa das Superoxid-Radikal in Immunzellen, wie Monozyten, Makrophagen und polymorphkernigen Leukozyten, durch membranständige NADPH-Oxidasen gebildet und in das extra-zelluläre Milieu abgegeben. In durch entzündliche Stimuli aktivierten Zellen steigt die normalerweise nur geringe Superoxid-Bildung um das Zehnfache an ("oxidative burst"). Die Bildung dieser Spezies ist nicht nur für die Abtötung der Bakterien verantwortlich, sondern dient auch der Rekrutierung von Leukozyten zum Entzündungsherd und hat somit eine Funktion zur Entzündungsverstärkung. Weiterhin greifen reaktive Sauerstoffspezies auf der Ebene der Transkription in die Bildung von Enzymen, z.B. NOS-II, ein. Sie aktivieren auch Transkriptionsfaktoren, wie z.B. die NF-κB-B-Familie und Proteinkinasen, während sie Protein-Tyrosin-Phosphatasen inaktivieren.

Auch die reaktive Stickstoffverbindung Stickstoffmonoxid wird enzymatisch und streng kontrolliert in einer Reihe von Geweben gebildet. Ausgangssubstanz ist die Aminosäure L-Arginin, aus der das freie Radikal durch das Enzym NO-Synthestase (NOS) gebildet wird. In Immunzellen, speziell in Makrophagen und Granulozyten, kann nach Stimulation die induzierbare NOS (iNOS) exprimiert werden. Stimuli sind dabei vor allem Auslöser von Entzündungsreaktionen, wie Bakterien oder deren Bestandteile, inflammatorische Zytokine etc.

Das Quenchen und Abfangen solcher reaktiven Sauerstoff- und Stickstoffspezies kann die Reaktionskette unterbrechen und so regulatorisch auf Transkriptions- und Enzymaktivierungsebene wirken. Zusätzlich können Gewebeschäden, wie z.B. Lipidperoxidationen, durch reaktive Sauerstoffspezies vermieden werden.

Eine prophylaktische oder therapeutische Wirkung erfolgt somit auf verschiedenen Ebenen des Prozesses.

So werden durch die Ausbildung einer Art Schutzschicht z.B. mechanischen Reizungen oder der Kontakt mit Allergenen oder Bakterien vermieden. Die erfindungsgemäße Zusammensetzung enthält daher bevorzugt entsprechend z.B. lipophile und Gel-bildende Komponenten, die einen befeuchtenden, pflegenden Film auf Epithelien, wie etwa der Cornea, ausbilden können.

Weiterhin greifen Komponenten der beschriebenen modulatorhaltigen Zusammensetzung auf einer oder mehreren Ebenen, z.B. Gentranskription, qualitative und/oder quantitative Modulation der Freisetzung von Mediator-Molekülen, Modulation der Signaltransduktion, etc., in z.B. den Prozess der Entzündungsentstehung und/oder Verstärkung regulierend ein.

Durch die Auswahl und die Zusammensetzung der ω-3-Fettsäuren in der pharmazeutischen Zusammensetzung kann die Entstehung entzündungshemmender Mediatoren begünstigt werden, um prophylaktisch oder therapeutisch Störungen und Erkrankungen vorzubeugen. Bei-spiele für Erkrankungen sind Irritationen, Reizung und Schwellung der Schleimhäute, Augenbrennen, Wundheilung, Behandlung des Keratokonjunktivitis sicca, des Sjörgen-Syndroms.

In einer bevorzugten Zusammensetzung ist der mindestens eine Modulator ein Inhibitor oder Antagonist des NF-κB-Transkriptionsfaktors und bevorzugt ausgewählt
- aus natürlichen Quellen, insbesondere aus der Gruppe bestehend aus Allicin, Curcumin, EGCD, Genistein, Melatonin, Quercetin, Resveratrol, Silymarin, Sulphoraphanen oder Mischungen hieraus, und/oder
- aus der Gruppe bestehend aus synthetischen Inhibitoren, insbesondere Pyrrolidin-dicarbamat, 2-Chlor-4-(trifluormethyl)pyrimidin-5-N-(3',5'-bis(trifluor-methyl)phenyl)-carboxamid und/oder Mischungen hieraus.

Weitere Beispiele für Modulatoren der NF-kB-Aktivie-rung (Antagonisten und/oder Inhibitoren) aus natürlichen Quellen sind: alpha-Liponsäure (Thioctsäure) und Dihydroliponsäure, 2-Amino-1-methyl-6-phenylimidazol (4,5 b]pyridine (PhIP), N-acetyldopamindimere (from P. cicadae), Allopurinol, Anetholdithiolthione, Apocynin, Aretemsia p7F (5,6,3',5'-tetramethoxy 7,4'-hydroxyflavone), Astaxanthin, Autumn olive extracts; olive leaf extracts, Avenanthramide (from oats), Bamboo culm extract, Benidipin, Bis-eugenol, Bruguiera gymnorrhiza compounds, butyliertes Hydroxyanisol (BHA), Cepharanthin, Caffeic Acid Phenethyl Ester (3,4-dihydroxycinnamic acid, CAPE), Carnosol, Carotinoide (z.B. beta-Carotin), Carvedilol, Catechol Derivatives, Centaurea L (Asteraceae) extracts, Chalcone, Chlorogenic acid, 5-chloroacetyl-2-amino-1,3-selenazoles, Cholestin, Chroman-2-carboxylic acid N-substituted phenylamides, polyphenols for example from Cocoa or Crataegus pinnatifida, Coffee extract (3-methyl-1,2-cyclopenta-nedione), Curcumin (Diferulolylmethan); dimethoxycur-cumin; ER24 anlog, Dehydroepiandrosterone (DHEA) und DHEA-Sulfat (DHEAS), Dibenzylbutyrolactone lignans, Diethyldithiocarbamat (DDC), Diferoxamin, Dihydroisoeugenol; Isoeugenol; Epoxypseudoisoeugenol-2-methylbutyrat, Dihydrolipoic Acid, Dilazep + feno-fibric acid, Dimethyldithiocarbamates (DMDTC), Disulfiram, Edaravone, EPC-K1 (phosphodiester compound of vitamin E and vitamin C) Epigallocalechin-3-gallate (EGCG; green tea polyphenols), Ergothioneine, Ethylene Glycol Tetraacetic Acid (EGTA), Eupatilin, Fisetin, Flavonoids (Crataegus; Boerhaavia diffusa root; xanthohumol; Eupatorium arnottianum; genistein; kaempferol; quercetin, daidzein; flavone; isorhamnetin; naringenin; pelargonidin; finestin; Sophora flavescens; Seabuckthorn fruit berry), Sesquiterpenlactone wie z.B. Helenalin z.B aus Arnika-Extrakten, Folic acid, Gamma-glutamylcysteine synthetase (gamma-GCS), Ganoderma lucidum polysaccharides, Garcinol (from extract of Garcinia indica fruit rind), Ginkgo biloba extract, Glutathione, Guaiacol (2-methoxyphenol), Hematein, Hinokitiol, Hydroquinone, 23-hydroxyursolic acid, IRFI 042 (Vitamin E-like compound), Iron tetrakis, Isoflavone, Isosteviol, Isovitexin, Isoliqui-ritigenin, Kallistatin, Kangen-karyu extract, L-cysteine, Lacidipine, Lazaroids, Ligonberries, Lupeot, Lutein, Magnolol, Maltol, Melatonin, Extract of the stem bark of Mangifera indica L., 21(alpha, beta)-methylmelianodiol, 21(alpha,beta)-methylmeli-anodiol, Mulberry anthocyanins, N-acetyl-L-cysteine (NAC), Nacyselyn (NAL), Nordihydroguaiaritic acid (NDGA), Ochnaflavone, Onion extract (2,3-dihydro-3,5-dihydroxy-6-methyl-4H-pyranone), Orthophenanthroline, N-(3-oxo-dodecanoyl)homoserine lactone, N-(3-oxo-dodecanoyl)homoserine lactone, Paricalcitol, Parthe-nolid, ein Sesquiterpenlacton, Terpene und ein Sesquiterpenlacton, Parthenolid, Phenolic antioxidants (Hydroquinone and tert-butyl hydroquinone), alkenylphenols from Piper obliquum, alpha-phenyl-n-tert-butyl-njtrone (PBN), Phenylarsine oxide (PAO, tyrosine phosphatase inhibitor), Phyllanthus urinaria, Phytosteryl ferulates (rice bran), Piper longum Linn. Extract, Pitavastatin Prodelphinidin B2 3,3'di-O-gallate, Pterostilbene, Pyrrolinedithiocar-bamate (PDTC), Quercetin, Ref-1 (redox factor 1), Ref-1 (redox factor 1), Rotenone, Roxithromycin, Rutin, S-allyl-cysteine (SAC, garlic compound), Salo-gaviolide (Centaurea ainetensis), Sauchinone, Silybin, Spironolactone, Taxifolin, Tempol, Tepoxaline (5-(4-chlorophenyl)-N-hydroxy(4-methoxyphenyl)-N-methyl-1H-pyrazole-3-propanamide), Thymoquinone, Tocotrienol (palm oil), Vanillin (2-hydroxy-3-methoxybenzaldehyde), Vitamin B6, a-torphryl succinate, a-torphryl succinate, 2-torphryl acetate, PMC (2,2,5,7,8-pentamethyl-6-hydroxychromane), Yakuchinone A and B, Zerumbon aus Zingiberarten (Ingwer).

Als weitere Beispiele für synthetische Quellen sind folgende zu nennen: Kortisone und Glukokortikoide, sowie deren Ester, z.B. 16α,17-[(R)-Cyclohexylmethy-lendioxy]-11β,21-dihydroxypregna-1,4-dien-3,20-dion-21-isobutyrat), Salicylanilid-Inhibitoren, 3,4-dihydro-1,1-dimethyl-2H-1, 2-benzoselenazine; Declopramide und Dexlipotam, N-(-Acetylphenyl)-2-hydroxy-5-iodphenyl-carboxamid; N-(-2,4-Difluoro-phenyl)-2-hydroxy-5-nitrophenylcarboxamid; N-(2,4-Difluorphenyl)-2 hydroxy-5-iodphenylcarboxamid, oder ein pharmazeutisch annehmbares Salz, Hydrat oder Solvat davon. Es können auch zusätzlich Modulatoren der COX-2 enthalten sein, wie z.B. Basilikum, Berberine, Curcumin, EGCG, Ingwer, Hopfen (Humulus lupulus), Fischöl, Oregano, Quercetin, Resveratrol, Rosemarie.

Besonders bevorzugte Wirkstoffe, die in der erfindungsgemäßen ophthalmologischen Zusammensetzung eingesetzt werden sind entzündungshemmende Stoffe, wie Acetylsalicalsäure und Derivate, z.B als L-Lysin, antiseptische Stoffe, wie Biprocathol, Antibiotika wie Ampicillin, Sulfacetamid, Doxycyclin, Gentamycin oder Ciprofloxacin, Antiallergika wie Cromoglicinsäure, Antihistaminika wie Levocabastin, Azelastin und/oder Dexpanthenol.

Bevorzugte Applikationsmöglichkeiten der erfindungsgemäßen Zusammensetzung sind dabei die topische Applikation am Auge, insbesondere durch Einträufeln oder Eintropfen ins Auge bzw. auf die Augenoberfläche, Einsprühen oder Aufsprühen ins Auge bzw. auf die Augenoberfläche und/oder durch Einbringen auf die Innenseite des unteren Augenlids bzw. in den Bindehautsack.

Die ophthalmologische Zusammensetzung gemäß der Erfindung kann1 mal täglich bis 4 mal stündlich, bevorzugt 2 mal täglich bis 12 mal täglich appliziert werden.

Die erfindungsgemäße ophthalmologische Zusammensetzung eigne sich insbesondere zur Verbesserung der Wundheilung nach chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen, und Eingriffen an der Bindehaut.

Die Erfindung betrifft zudem einen Fluidspender für ein keimfreies Fluid, mit
a) einem Durchgang, der eine Einlassöffnung für ein in einem Vorratsbehälter aus einem flexiblen Material enthaltenes Fluid und eine Ausströmöffnung zum Ausgeben des Fluids verbindet und darin wenigstens eine oligodynamisch aktive Substanz besitzt, die mit dem Fluid in Kontakt ist;
b) einer Dosierpumpe, die ohne Luftdruckkompensation arbeitet, umfassend ein Einlassventil zum Schließen der Einlassöffnung, wobei das Einlassventil ein Material aufweist, das mit dem Fluid über eine oligodynamisch aktive Substanz wechselwirken kann; und
c) eine Federeinrichtung, die in Kontakt mit dem Fluid steht,
   wobei das Einlassventil und die Federeinrichtung ein rostfreies Stahlmaterial als eine oligodynamisch aktive Substanz aufweisen und eine Dekontaminierungseinrichtung im oberen Teil des Auslasskanals vorgesehen ist, wobei die Dekontaminierungseinrichtung ein Material aufweist, das mit dem Fluid über eine oligodynamische Substanz wechselwirken kann, die ausgewählt ist aus der Gruppe bestehend aus Silber, Silbersalzen, anderen Silberverbindungen, Legierungen und Nanomeren davon in entweder metallischer oder Salzform oder als eine chemische Verbindung daraus, wobei das im Vorratsbehälter enthaltene Fluid eine erfindungsgemäße ophthalmologische Zusammensetzung ist.

Bezüglich des Fluidspenders, in dem gemäß der Erfindung das ophthalmologische Vehikelsystem bevorratet werden kann, wird auf die Patentanmeldung EP 1466 668 A1 verwiesen. Sämtliche Ausführungsformen bezüglich des Fluidspenders dieser Patentanmeldung werden durch Bezugnahme auch zum Gegenstand dieser Patentanmeldung gemacht.

Erfindungsgemäß wird ebenso ein Kosmetikum bzw. die Verwendung einer Zusammensetzung als Kosmetikum angegeben, wobei die Zusammensetzung enthält:
a) mindestens eine ω-3-Fettsäure und/oder ein ω-3-Fettsäurederivat ausgewählt aus der Gruppe bestehend aus Lipiden, Oxygenierungsprodukten, Carboxylatsalzen, Estern, Mono-, Di- oder Triglyceriden, Amiden, sonstigen pharmakologisch akzeptablen Carbonsäurederivaten und Mischungen hiervon, sowie
b) mindestens ein Glucosaminoglucan, hiervon abgeleitete pharmakologisch akzeptable Derivate, sowie Mischungen hiervon.

Bevorzugte Ausführungsformen der als Kosmetikum verwendete Zusammensetzung entsprechen den oben beschriebenen Ausführungsformen der erfindungsgemäßen ophthalmologischen Zusammensetzung.

Besonders bevorzugt eignet sich diese Zusammensetzung als Gesichtskosmetikum, insbesondere im Bereich der Augen, Augenlider, Bereich um die Augen und Anwendung im Naseninnern (Reinigung und Pflege der Nasenschleimhaut).

Die vorliegende Erfindung wird anhand der nachfolgenden beispielhaften Formulierungen näher erläutert, ohne die Erfindung auf die dort dargestellten speziellen Parameter zu beschränken.

Die folgenden beispielhaften erfindungsgemäßen Rezepturen eignen sich als ophthalmologische Zusammensetzung zur Prophylaxe und/oder Behandlung von strukturellen Epithelschäden, Schürfungen, Abrasionen, Verletzungen, Reizungen, Entzündungen, Verätzungen und Schäden hervorgerufen durch Benetzungsstörungen aus der Gruppe, bestehend aus Sjögren-Syndrom, Sicca-Syndrom, Benetzungsstörungen des Auges bei Kontaktlinsenträgern, Lidrandentzündung (Blepharitis) und/oder chirurgischen Eingriffen am Auge, zur Förderung oder Unterstützung der Wundheilung des Augenepithels und/oder zur Förderung der Proliferation der Epithelzellen des Auges; ebenso sind diese Rezepturen als Kosmetika verwendbar.

### Beispiel 1- Öl-in-Wasser-Emulsion

| **Bestandteile** | **Menge (Gew.-%)** |
|---|---|
| Algenöl | 0,1-16% |
| Bibrocathol | 3 % |
| Mannitol | 4,5% |
| Polysorbat 80 | 0,2-16% |
| EDTA | 0,05% |
| Natriumacetat | 0,03% |
| Essigsäure | 0,04% |
| Vitamin E | 0,075 % |
| Hyaluronsäure | 0,1% |
| Wasser | ad 100 |

### Beispiel 2 - Öl-in-Wasser-Mikroemulsion

| Bestandteile | Menge (Gew.-%) |
|---|---|
| Hyaluronsäure | 0,1-1 |
| Magrogol-25-cetostearylether (=Cremophor A25) | 2-5 |
| Eicosapentaensäure/ Docosahexaensäure-Mischung (je als Ethylester) | 0,1 |
| Q10 | 0,05 |
| Reservatrol | 0,01-1 |
| Citronensäure | 0,005 |
| Natriumcitrat | 0,85 |
| Sorbit | 3,2 |
| Wasser | ad 100 |

### Beispiel 3 - Öl-in-Wasser-Nlikroemulsion

| **Bestandteile** | **Menge (Gew.-%)** |
|---|---|
| Hyaluronsäure | 0,1-1 |
| Magrogolglycerol-ricinoleat (=Cremophor EL) | 0,1-5 (bevorzugt 0,3-1%, besonders bevorzugt 0,5-0,8%) |
| Eicosapentaensäure/Docosahexaensäure-Mischung (je als Ethylester) | 0,005-0,5 |
| Gemischte Tocopherole | <0,3 |
| Gegebenfalls Q10 (und/oder Taurin und/oder Ascorbylpalmitat und/oder Reservatrol) | Ggf. 0,005-1 |
| Citronensäure | 0,005 |
| Natriumcitrat | 0,85 |
| Sorbit | 3,2 |
| Wasser | ad 100 |

### Beispiel 4 - Öl-in-Wasser-Mikroemulsion

| **Bestandteile** | **Menge (Gew.-%)** |
|---|---|
| Hyaluronsäure | 0,1-1 |
| Magrogolglycerol-ricinoleat (=Cremophor EL) | 0,1-5 (bevorzugt 0,3-1%, besonders bevorzugt 0,5-0,8%) |
| Eicosapentaensäure/Docosahexaensäure-Mischung (je als Ethylester) | 0,1-0,5 |
| Castoröl | 0,1-0,5 |
| Gemischte Tocopherole | <0,3 |
| Citronensäure | 0,005 |
| Natriumcitrat | 0,85 |
| Sorbit | 3,2 |
| Wasser | ad 100 |

### Beispiel 5 - Öl-in-Wasser-Mikroemulsion

| **Bestandteile** | **Menge (Gew.-%)** |
|---|---|
| Hyaluronsäure | 0,1-1 |
| PEG-20_glyceryl-stearate (=Cutina 24) | 1 |
| Eicosapentaensäure/ | |
| Docosahexaensäure-Mischung (je als Ethylester) | 0,1 |
| Gemischte Tocopherole | <0,3 |
| Gegebenfalls Q10 (und/oder Taurin und/oder Ascorbylpalmitat und/oder Reservatrol) | Ggf. 0,005-1 |
| Citronensäure | 0,005 |
| Natriumcitrat | 0,85 |
| Sorbit | 3,2 |
| Wasser | ad 100 |

### Beispiele6 bis 9 - Weitere Formulierungen als Emulsionen

| | **Bsp. 5** | **Bsp. 6** | **Bsp. 7** | **Bsp. 8** |
|---|---|---|---|---|
| | mg/ml (falls nicht anders angegeben) | | | |
| Heparin-Natrium | 1.300I.E/mg | | | |
| Hyaluronsäure, Natriumsalz | 0,1-1 | 0,01-0,2 | 0,1-1 | 0,25 |
| Sorbit | | 32 | | 20 |
| Glycerol 85 % | 17,6 | | | |
| Dexpanthenol | | | 20 | 20 |
| Algenöl | 0,1-0,3 | 0,05-0,3 | | |
| Perillaöl | | | 0,01-1 | 0,01-0,5 |
| Lecithin | 0,1-3 | 0,001-0,03 | | 0,005-0,5 |
| Isländisch Moos-Extrakt | | | | 0,05-2 |
| Ubichinon (Q10) | | 0,005-0,05 | | 0,005-0,05 |
| Vitamin E | 0,005-0,5 | | | 0,005-0,5 |
| Honig | | | | 0,05-0,2 |
| Tween 80 | | 0,0005-0,1 | | 0,005-0,5 |
| Natriumcitat x 2 H2O | 8,5 | 8,5 | 14,9 | |
| Citronensäure wasserfrei | 0,05 | 0,05 | 0,63 | |
| Natriumdihydrogenphosphat-Dihydrat | | | | 2,9 |
| Natriumhydrogenphosphat x 2 H₂O | | | | 1,05 |
| Wasser | ad 1 ml | | | |

Die genannten Beispiele können mittels NaCl in den Osmolaritäten vereint werden. Bevorzugt ist eine Osmolarität von 280 bis 330 mOsmol.

### Untersuchungen zur Stabilität der Zusammensetzungen

Die nachfolgenden Untersuchungen belegen die Lagerstabilität der erfindungsgemäßen ophthalmologischen Zusammensetzungen. Dabei wurden Mikroemulsionen (Öl-in-Wasser) untersucht

### Verwendete Substanzen:

| **Substanz** | **Hersteller** | **Batch / LOT/Charge** |
|---|---|---|
| HyloComod, 0,1% | Ursapharm | 101.958.203.124 |
| Q10 | Fagron | 10H19N10 |
| Macrogolglycerolricinoleat | BASF | 07361816K0 |
| DHA/EPA | KD Pharma | FE11182 |
| MilliQ, autoklaviert | Eigene Herstellung | |

### Durchführung:

### Herstellung der Mikroemulsionen:

Es wurden vier Ansätze mit unterschiedlichen Macrogolglycerolricinoleat Konzentrationen hergestellt:

| **Ansatz** | **Hylo- Comod** | **Macrogolglycerol ricinoleate** | **DHA/EPA** | **Q10** | **Insgesamt** |
|---|---|---|---|---|---|
| | [%] | [%] | [%] | [%] | [%] |
| **1** | 98.60 | 1.25 | 0.1 | 0.05 | 100.00 |
| **2** | 98.85 | 1.00 | 0.1 | 0.05 | 100.00 |
| **3** | 99.10 | 0.75 | 0.1 | 0.05 | 100.00 |
| **4** | 99.35 | 0.50 | 0.1 | 0.05 | 100.00 |

In folgender Reihenfolge wurden Macrogolglycerolricinoleat, DHA/EPA und Q10 in 50mL Bechergläser eingewogen und bei 60°C auf dem Magnetrührer bis zur Homogenität gerührt.

Die Macrogolglycerolricinoleat-DHA/EPA-Q10-Mischung wurden wie folgt in die vorgewärmten 500mL Messzylinder eingewogen:
Zugabe von jeweils 380mL HyloComod in 80-100mL Schritten und Rühren im Wasserbad bei 60°C bis zum vollständigem Lösen. Bei unvollständigem Lösen wurde mit dem Glas-Rührstab gelöst. Nachdem 380mL HyloComod zugegeben wurde, wurde die Temperatur für 3min gehalten. Abkühlen der Ansätze auf der Arbeitsplatte auf RT. Nach Abkühlen wurden die Ansätze mit HyloComod auf 400mL aufgefüllt.

### Filtration:

### Waschen der Fluorodyne II Filter mit autoklaivertem MilliQ.

Filtration der Ansätze mit den Fluorodyne II Filtern und der 50mL Spritzen mithilfe der peristaltischen Pumpe (max. 20rpm) in die 500mLSchottflaschen, dabei wurden jeweils 50mL Erstfiltrat verworfen.

Nach der Filtration wurden die Reagenzröhrchen mit jeweils 5mLdes klaren Filtrat befüllt.

Pro Lagertemperatur wurden 3-9 Reagenzröhrchen als Parallelen mit den in der nachfolgenden Tabelle aufgelisteten Probennummern geführt:

| **Ansatz** | **5°C** | | **40°C** | | **+5/40 °C** | | **5 °C** | | **RT** | | **Summe** |
|---|---|---|---|---|---|---|---|---|---|---|---|
| 1 | 6 | 4-9 | 6 | 10-15 | 6 | 16-21 | 3 | 22-24 | 3 | 25-27 | 27 |
| 2 | 6 | 31-36 | 6 | 37-42 | 6 | 43-48 | 3 | 49-51 | 3 | 52-54 | 27 |
| 3 | 6 | 58-63 | 6 | 64-69 | 6 | 70-75 | 3 | 76-78 | 3 | 79-81 | 27 |
| 4 | 6 | 85-90 | 6 | 91-96 | 6 | 97-102 | 3 | 103-105 | 3 | 106-108 | 27 |

### Ergebnisse:

Abfülltag 21.02.2012; Tag 0

### Lagerbedingungen:

RT: Raumtemperatur
5°C: Kühlschrank, bei 5°C
40°C: Brutschrank, bei 40°C und bei einer relativen Luftfeuchtigkeit von 75 r.H.%
5°C/40°C: Wechsel der Proben zwischen Kühlschrank (5°C) und Brutschrank (40°C und mit einer relativen Luftfeuchtigkeit von 75 r.H.% t) im 24h Takt.

### 5°C; 5°C/40°C; 40°C und RT-Proben:

Die Proben wurden am Tag 0 bei den jeweiligen Temperaturen gelagert. Die Proben vom Schaukeltest (14.G. ICH Guidelines for stability tests) stehen im Wechsel ca. 24Stunden bei 5°C bzw. 40°C bei einer relativen Luftfeuchtigkeit von 75 r.H.%. Anfangs (bis Tag 35) wurden die Proben im Abstand von 7 Tagen und am Tag 63 fotografiert. Die Proben sind bisher klar und zeigen keine Veränderung, weder durch die verschiedenen Lagerbedingungen noch durch die verschiedenen Emulgatorkonzentrationen.

Die mit den Proben erzielten Versuchsergebnisse sind in den nachfolgenden Tabellen wiedergegeben.

| **Raumtemperatur** | | | | | | | | | |
|---|---|---|---|---|---|---|---|---|---|
| **Ansatz** | **Emulgator-Konzentration (%)** | **Proben** | **Tag 0** | **Tag 7** | **Tag 14** | **Tag 21** | **Tag 28** | **Tag 35** | **Tag 63** |
| **1** | **1,25** | 25-27 | klar | klar | klar | klar | klar | klar | klar |
| **2** | **1,00** | 52-54 | klar | klar | klar | klar | klar | klar | klar |
| **3** | **0,75** | 79-81 | klar | klar | klar | klar | klar | klar | klar |
| **4** | **0,5** | 106-108 | klar | klar | klar | klar | klar | klar | klar |
| | | | | | | | | | |

| **5°C** | | | | | | | | | |
|---|---|---|---|---|---|---|---|---|---|
| **Ansatz** | **Emulgator-Konzentration (%)** | **Proben** | **Tag 0** | **Tag 7** | **Tag 14** | **Tag 21** | **Tag 28** | **Tag 35** | **Tag 63** |
| **1** | **1,25** | 4-9; 22-24 | klar | klar | klar | klar | klar | klar | klar |
| **2** | **1,00** | 31-36;49-51 | klar | klar | klar | klar | klar | klar | klar |
| **3** | **0,75** | 58-63;76-78 | klar | klar | klar | klar | klar | klar | klar |
| **4** | **0,5** | 85-90; 103-105 | klar | klar | klar | klar | klar | klar | klar |
| | | | | | | | | | |

| **40°C (75r.H.%)** | | | | | | | | | |
|---|---|---|---|---|---|---|---|---|---|
| **Ansatz** | **Emulgator-Konzentration (%)** | **Proben** | **Tag 0** | **Tag 7** | **Tag 14** | **Tag 21** | **Tag 28** | **Tag 35** | **Tag 63** |
| **1** | **1,25** | 10-15 | klar | klar | klar | klar | klar | klar | klar |
| **2** | **1,00** | 37-42 | klar | klar | klar | klar | klar | klar | klar |
| **3** | **0,75** | 64-69 | klar | klar | klar | klar | klar | klar | klar |
| **4** | **0,5** | 91-96 | klar | klar | klar | klar | klar | klar | klar |
| | | | | | | | | | |

| **5°C/40°C (75r.H.%)** | | | | | | | | | |
|---|---|---|---|---|---|---|---|---|---|
| | | | | | | | | | |

| **Ansatz** | **Emulgator-Konzentration (%)** | **Proben** | **Tag 0** | **Tag 7** | **Tag 14** | **Tag 21** | **Tag 28** | **Tag 35** | **Tag 63** |
|---|---|---|---|---|---|---|---|---|---|
| **1** | **1,25** | 16-21 | klar | klar | klar | klar | klar | klar | klar |
| **2** | **1,00** | 43-48 | klar | klar | klar | klar | klar | klar | klar |
| **3** | **0,75** | 70-75 | klar | klar | klar | klar | klar | klar | klar |
| **4** | **0,5** | 97-102 | klar | klar | klar | klar | klar | klar | klar |

Die Versuche zeigen, dass auch bei Einsatz eines einzigen Emulgators sowie mit geringen Emulgatorkonzentrationen von bspw. 0,75 Gew.-% oder 0,5 Gew.-% stabile Mikroemulsionen erhältlich sind.

### Untersuchungen zur pharmakologischen Wirksamkeit der Zusammensetzungen

Die nachfolgenden Experimente belegen das Proliferationsvermögen auf das Augenepithel und somit die ophthalmologische Wirksamkeit der erfindungsgemäßen Zusammensetzungen.

### Versuchsbeschreibung:

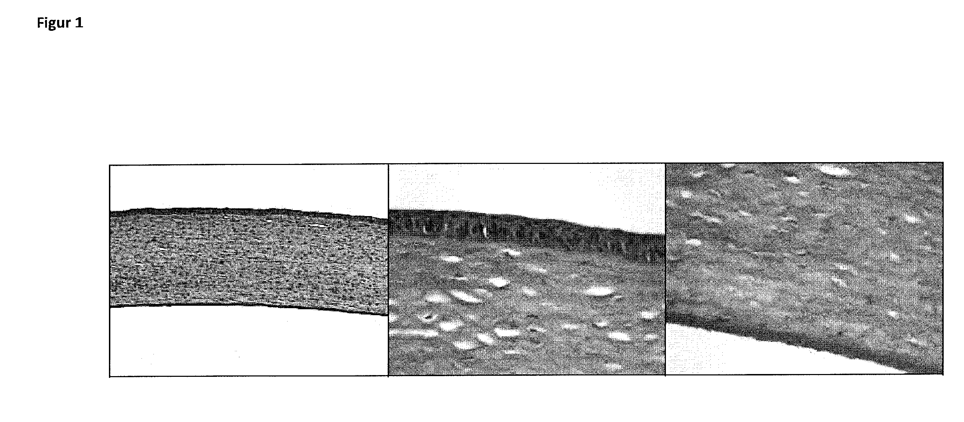

Das Epithel eines frisch enukleierten Kaninchenbulbus wurde 8h post mortem bei 32° C und einer Luftfeuchtigkeit von 95 % für 60 min ganzflächig einem Volumen von 0,5 ml der zu untersuchenden Substanz ausgesetzt. Anschließend wurde die Substanz mit Ringer-Lösung abgespült, die Hornhautkulturen wurden teilweise in Formalin fixiert und nach Entwässerung in Paraffin eingebettet. Die Färbung der Paraffinschnitte erfolgte mittels Hämatoxylin-Eosin. Die Versuchsergebnisse sind als histologische Befunde in den Figuren 1 bis 5 abgebildet. Dargestellt sind jeweils von links nach rechts: Gesamtübersicht der Hornhaut in 16-facher Vergrößerung, Hornhautepithel in 400-facher Vergrößerung und Hornhautendothel in 400-facher Vergrößerung.

Die eingesetzten Substanzen sind dabei:

### Kulturmedium:

Ringerlösung: Die Ringer-Lösung ist eine isotone Elektrolytlösung, die u.a. als Nährmedium für Frischgewebe verwendet wird. Die Standard-Ringerlösung enthält auf 1000 ml Aqua destillata 8,6 g Natriumchlorid, 0,3 g Kaliumchlorid, 0,33 g Calciumchlorid.

### Hylo-COMOD^{®}:

HYLO-COMOD^{®} ist eine sterile, konservierungsmittelfreie Lösung mit 1 mg/ml Natriumhyaluronat, einem Citratpuffer, Sorbitol und Wasser und wird von der URSAPHARM Arzneimitel GmbH vertrieben.

### Ursapharm A:

Mikroemulsionsgel auf Wasser-in-Öl-Basis, enthaltend 7,5 Gew.-% Lecithin (Epicuron 200), 0,4 Gew.-% Wasser, 0,1 Gew.-% eines Gemischs aus Eicosapentaensäureethylester / Docosapentaensäureethylester (Gewichtsverhältnis ca. 73 : 27) sowie ad 100 Gew.-% Isopropylmyristat.

### Ursapharm C:

Mikroemulsionsgel auf Öl-in-Wasser-Basis, enthaltend 0,1 Gew.-% eines Gemischs aus Eicosapentaensäureethylester / Docosapentaensäureethylester (Gewichtsverhältnis ca. 73 : 27), 1 Gew.-% Magrogolglycerolricinoleat (z.B. Cremophor EL), 0,1 Gew.-% Hyaluronsäure, < 0,3 Gew.-% gemischte Tocopherole, 0,05 Gew.-% Q10, 3,2 Gew.-% Sorbit, 0,85 Gew.-% Natriumcitrat, 0,005 Gew.-% Citronensäure sowie ad 100 Gew.-% Wasser.

### Versuchsergebnisse

Die histologischen Ergebnisse der nachfolgenden Experimente sind in den Figuren 1 bis 5 dargestellt.

### Experiment 1: Negativkontrolle - lediglich Simulation des Lidschlages durch Applikation von Kulturmedium - Figur 1

Das Hornhautepithel besteht aus einer Basalzellschicht, etwa 2-3 Flügelzellschichten und einer geschlossenen Fläche aus Schuppenzellen, die die Hornhaut nach außen abschließen. Nach unten wird die Hornhaut von einer intakten Endothelzellschicht abgeschlossen. Zarte Ödembildung ist an den im Stroma sichtbaren Spalten zu erkennen. Im Wesentlichen entspricht dieses Bild dem morphologischen Befund einer gesunden Kaninchenhornhaut.

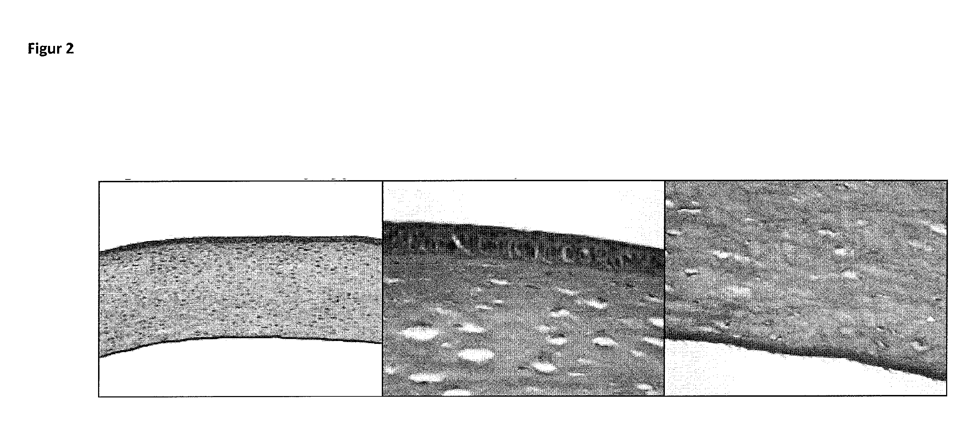

### Experiment 2: Negativkontrolle 2-Tropfapplikation alle 60 min, HYLOCOMOD^{®} - Figur 2

Das Hornhautepithel besteht aus einer Basalzellschicht, etwa 2-3 Flügelzellschichten und einer geschlossenen Fläche aus Schuppenzellen, die die Hornhaut nach außen abschließen. Typisch für die Applikation von Hyaluronat ist die etwas stärkere Anfärbbarkeit der Epithelien. Im Epithel gibt es ein winziges Ödem, erkennbar an den Aufhellungen zwischen den Zellen. Nach unten wird die Hornhaut von einer intakten Endothelzellschicht abgeschlossen (die hier durch ein Schnittartefakt leicht ausgefranzt erscheint). Dies entspricht dem morphologischen Befund einer gesunden Kaninchenhornhaut.

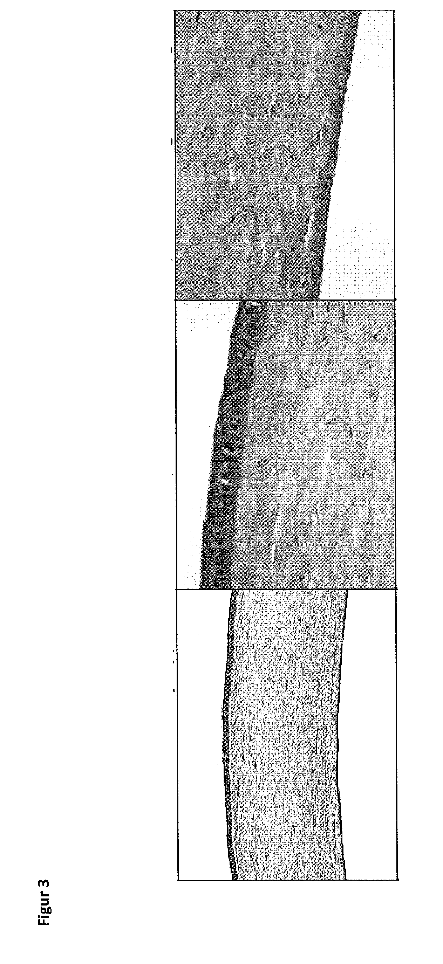

### Experiment 3: Positivkontrolle-Tropfapplikation alle 60 min, Benzalkoniumchlorid 0,001 % in Ringer-Lösung - Figur 3

Die Basalzellschicht erscheint deutlich aufgelockert. Die Flügelzellen liegen größtenteils nur noch einschichtig auf der Basalzellschicht auf und besitzen ebenfalls eine aufgelockerte Struktur. Es ist keine geschlossene Fläche aus Schuppenzellen erkennbar. Nach unten wird die Hornhaut von einer intakten Endothelzellschicht abgeschlossen. Das vordere Stroma ist azellulär und etwas verdichtet als Zeichen einer massiven Permeationsstörung. Das Epithel zeigt gegenüber einer gesunden Hornhaut sowie gegenüber den Negativkontrollen deutliche morphologische Veränderungen.

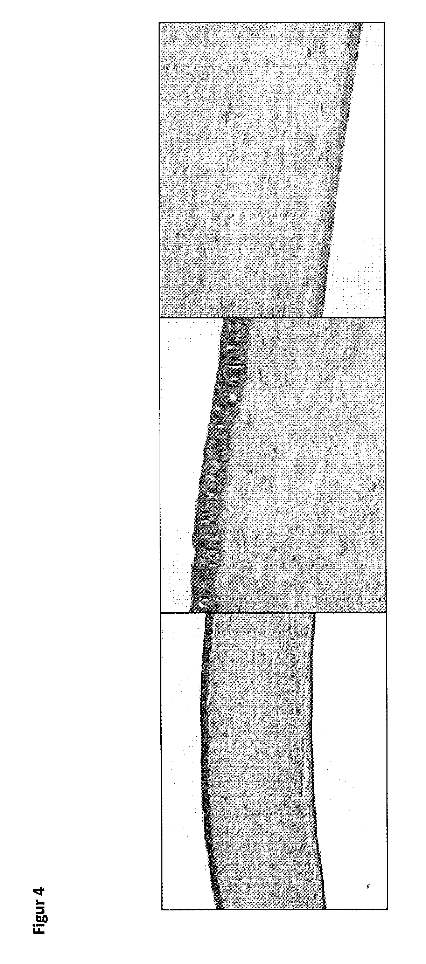

### Experiment 4: Testsubstanz - Tropfapplikation alle 60 min, Ursapharm A (Lecithin-Gel + IPM 0,4 %) - Figur 4

Die Basalzellschicht erscheint stark aufgelockert. Die Flügelzellen liegen größtenteils nur noch einschichtig auf der Basalzellschicht auf, stellenweise sind gar keine Flügelzellen mehr erkennbar. Eine geschlossene Fläche aus Schuppenzellen ist nur in kleinen Arealen auf der Hornhaut erkennbar. Es zeigen sich deutliche intraepitheliale Ödeme. Nach unten wird die Hornhaut von einer intakten Endothelzellschicht abgeschlossen. Das Epithel zeigt gegenüber einer gesunden Hornhaut sowie gegenüber den Positivkontrollen deutliche morphologische Veränderungen die in etwa der Positivkontrolle entsprechen.

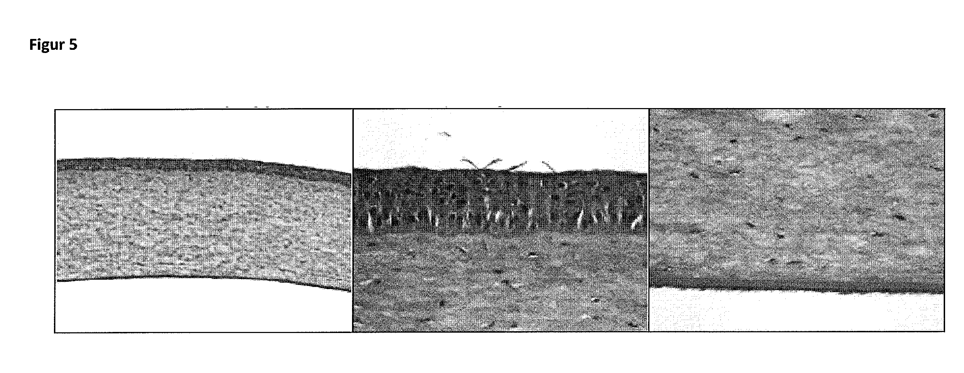

### Experiment 5: Testsubstanz - Tropfapplikation alle 60 min, Ursapharm C - Figur 5

Das Epithel besteht aus einer Basalzellschicht, etwa 4-5 Flügelzellschichten und einer geschlossenen Fläche aus Schuppenzellen, die die Hornhaut nach außen abschließen. Es scheint zu einer Epithelproliferation bzw. zu einer zarten Schwellung der Flügelzellen zu kommen, die deutlich über ihrem normalen Volumen zugenommen haben. Nach unten wird die Hornhaut von einer intakten Endothelzellschicht abgeschlossen (hier durch ein Schnittartefakt leicht ausgefranzt). In beiden histologisch untersuchten Kulturen lässt sich eine Steigerung der Anzahl der Flügelzellschichten unter der Applikation mit Ursapharm C ableiten. Die Hornhaut entspricht sonst dem morphologischen Befund einer gesunden Kaninchenhornhaut.

## Patentansprüche

1. Ophthalmologische Zusammensetzung, enthaltend
a) mindestens eine ω-3-Fettsäure und/oder ein ω-3-Fettsäurederivat ausgewählt aus der Gruppe bestehend aus Estern, Mono-, Di- oder Triglyceriden, Lipiden, Oxygenierungsprodukten, Carboxylatsalzen, Amiden, sonstigen pharmakologisch akzeptablen Carbonsäurederivaten und Mischungen hiervon, sowie
b) mindestens ein Glucosaminoglucan, hiervon abgeleitete pharmakologisch akzeptable Derivate, sowie Mischungen hiervon,
zur Prophylaxe und/oder Behandlung von strukturellen Epithelschäden, Schürfungen, Abrasionen, Verletzungen, Reizungen, Entzündungen, Verätzungen und Schäden hervorgerufen durch Benetzungsstörungen aus der Gruppe, bestehend aus Sjögren-Syndrom, Sicca-Syndrom, Benetzungsstörungen des Auges bei Kontaktlinsenträgern, Lidrandentzündung (Blepharitis) und/oder chirurgischen Eingriffen am Auge, zur Förderung oder Unterstützung der Wundheilung des Augenepithels und/oder zur Förderung der Proliferation der Epithelzellen des Auges.

2. Ophthalmologische Zusammensetzung nach Anspruch 1, **dadurch gekennzeichnet, dass** die mindestens eine ω-3-Fettsäure
a) als freie Fettsäure, ausgewählt aus der Gruppe bestehend aus α-Linolensäure, Stearidonsäure, Eicosatetraensäure, Eicosapentaensäure (EPA), Docosapentaensäure (DPA), Docosahexaensäure, Hexadecatrienoensäure, Eicosatrienoensäure, Heneicosapentaenoensäure, Tetracosapentaenoensäure, Tetracosahexaenoensäure, hiervon abgeleitete Oxygenierungsprodukte sowie Mischungen oder Kombinationen hieraus
b) in Form eines Esters eines organischen Alkohols, bevorzugt eines linearen oder verzweigten aliphatischen einwertigen Alkohols mit 1 bis 18 Kohlenstoffatomen, besonders bevorzugt als Methyl-, Ethyl-, n-Propyl-, i-Propyl-, n-Butyl-, i-Butyl-, t-Butylester enthalten ist und/oder
c) in Form eines pflanzlichen oder tierischen Öls enthalten ist, das neben der mindestens einen ω-3-Fettsäure noch mindestens eine ω-6-Fettsäure enthält, wobei das molare Verhältnis der ω-3-Fettsäure zur ω-6-Fettsäure von 100:1 bis 1:100, bevorzugt 20:1 bis 1:10, weiter bevorzugt 15:1 bis 1:1, besonders bevorzugt von 8:1 bis 2:1 beträgt, vorliegt.

3. Ophthalmologische Zusammensetzung nach einem der vorhergehenden Ansprüche, **dadurch gekennzeichnet, dass** die mindestens eine ω-3-Fettsäure in Form eines Öls, ausgewählt aus der Gruppe bestehend aus Algenöl, Fischöl, Perillaöl, Shiöl, Leinöl, Leindotteröl, Sacha Inchi Öl, Rapsöl, Olivenöl, Nachtkerzenöl, Sojaöl, Hanföl, Walnussöl, Erdnussöl, Sesamöl, Maiskeimöl, Flachssamenöl und/oder Mischungen hieraus vorliegt.

4. Ophthalmologische Zusammensetzung nach einem der vorhergehenden Ansprüche, **dadurch gekennzeichnet, dass** der Gehalt der mindestens einen ω-3-Fettsäure und/oder des Derivats hiervon, bezogen auf die gesamte Zusammensetzung, zwischen 0,01 und 60 Gew.-%, bevorzugt zwischen 0,05 und 30 Gew.-%, besonders bevorzugt zwischen 0,1 und 5 Gew.-% beträgt.

5. Ophthalmologische Zusammensetzung nach einem der vorhergehenden Ansprüche, **dadurch gekennzeichnet, dass** das mindestens eine Glucosaminoglucan und/oder das hiervon abgeleitete Derivat ausgewählt ist aus der Gruppe bestehend aus Hyaluronsäure, Hyaluronaten, insbesondere Natriumhyaluronat, Chondroitinsulfat, Chondroitin, Keratinsulfat, Heparin, Heparinsulfat und/oder Mischungen hiervon.

6. Ophthalmologische Zusammensetzung nach einem der vorhergehenden Ansprüche, **dadurch gekennzeichnet, dass** der Gehalt des mindestens einen Glucosaminoglucans und/oder des hiervon abgeleiteten Derivats, bezogen auf die gesamte Zusammensetzung, von 0,001 bis 10 Gew.-%, bevorzugt von 0,005 Gew.-% bis 5 Gew.-%, besonders bevorzugt von 0,01 Gew.-% bis 1 Gew.-%, beträgt.

7. Ophthalmologische Zusammensetzung nach einem der vorhergehenden Ansprüche, **dadurch gekennzeichnet, dass** das mindestens eine Glucosaminoglucan und/oder das hiervon abgeleitete Derivat ein Molekulargewicht (Mw) von 30.000 Dalton bis 10.000.000 Dalton, bevorzugt 250.000 bis etwa 5.000.000 Dalton aufweist.

8. Ophthalmologische Zusammensetzung nach einem der vorhergehenden Ansprüche in Form einer Wasser-in-Öl-Emulsion, einer Öl-in-Wasser-Emulsion, einer Lösung, wässrigen Lösung einer Suspension, einer Nanosuspensionen, einer Emulsion, einer Nanoemulsion, eines Gels, eines thixotropen Gels, eines Lipogels, eines Organogels, eines Mikroemulsionsgels, eines Hydrogels, eines Sprühgels, einer Paste, einer Creme, einer Salbe, eines in situ Gels oder eines Aerosols.

9. Ophthalmologische Zusammensetzung nach einem der vorhergehenden Ansprüche in Form einer Öl-in-Wasser-Emulsion, insbesondere einer ÖI-in-Wasser-Mikroemulsion, oder einer Wasser-in-Öl-Emulsion, insbesondere einer Wasser-in-Öl-Mikroemulsion, enthaltend einen Emulgator oder eine Kombination aus einem oder mehreren Emulgatoren, wobei im Falle einer Öl-in-Wasser-Emulsion oder Öl-in-Wasser-Mikroemulsion der Emulgator oder jeder der Emulgatoren der Kombination oder die Mischung der Emulgatoren bevorzugt einen HLB-Wert > 10 aufweist.

10. Ophthalmologische Zusammensetzung nach vorhergehendem Anspruch, **dadurch gekennzeichnet, dass** der Emulgator
a) für die Öl-in-Wasser-Emulsion, insbesondere für die Öl-in-Wasser-Mikroemulsion ausgewählt ist aus der Gruppe der ethoxylierten Castoröle und oder ethoxylierten hydrogenierten Castoröle, bevorzugt Magrogolglycerolricinoleat (z.B. Cremophor EL), Macrogolglycerolhydroxystearat (z.B. Cremophor RH40), Glyceryl stearate (z.B. Cutina 24 (PEG-20-Glyceryl stearat)) und/oder Macrogolcetylstearylether (z.B. CremophorA25) und in einer Menge von 0,01 bis 5 Gew.-%, bevorzugt von 0,05 bis 1 Gew-%, besonders bevorzugt von 0,1 bis 0,9 Gew.-%, insbesondere von 0,1 bis 0,8 Gew.%, bezogen auf die gesamte Zusammensetzung enthalten ist, oder
b) für die Wasser-in-Öl-Emulsion, insbesondere für die Wasser-in-Öl-Mikroemulsion ausgewählt ist aus der Gruppe der Lecithine, bevorzugt Epikuron 200 und in einer Menge von 1 bis 10 Gew.-%, bevorzugt von 1 bis 7,5 Gew.-% bezogen auf die gesamte Zusammensetzung enthalten ist.

11. Ophthalmologische Zusammensetzung nach einem der Ansprüche 9 bis 10, **dadurch gekennzeichnet, dass** zusätzlich zum einen Emulgator oder eine Kombination aus mehreren Emulgatoren mindestens ein Coemulgator enthalten ist, vorzugsweise PEG 400 und/oder 1,2-Propylenglykol.

12. Ophthalmologische Zusammensetzung nach einem der vorhergehenden Ansprüche, enthaltend Silberionen in einer Konzentration von < 10 ppm, bevorzugt von 1 ppb bis 2 ppm, weiter bevorzugt von 10 ppb bis 1 ppm.

13. Ophthalmologische Zusammensetzung,
a) nach vorhergehendem Anspruch, **dadurch gekennzeichnet, dass** die Zusammensetzung bis auf Silberionen frei von Konservierungsmitteln ist oder
b) nach einem der vorhergehenden Ansprüche, **dadurch gekennzeichnet, dass** mindestens ein in der Ophthalmologie gebräuchliches Konservierungsmittel in einer Menge von 0,001 - 1 Gew.-%, vorzugsweise 0,01-0,5 Gew.-%, bevorzugt 0,01-0,04 Gew.-%, bezogen auf die gesamte Zusammensetzung, vorzugsweise ein Konservierungsmittel ausgewählt aus der Gruppe bestehend aus Polyquad, Natriumperborat, Purite; Alkohole, wie Chlorobutanol, Benzylalkohol, Phenoxyethanol; Carboxylsäuren wie Sorbinsäure; Phenole, wie Methly-/ Ethylparaben; Amidine wie Chorhexidindigluconat; quaternäre Ammoniumverbindungen wie Benzalkoniumchlorid, Benzethoniumchlorid und Cetylpyridiniumchlorid, Benzylbromid und/oder Kombinationen hieraus, oder
c) nach einem der Ansprüche 1 bis 11, **dadurch gekennzeichnet, dass** die Zusammensetzung frei von Konservierungsmitteln ist.

14. Ophthalmologische Zusammensetzung nach einem der vorhergehenden Ansprüche, insbesondere nach Anspruch 12 oder 13, enthaltend mindestens einen Komplexbildner, ausgewählt aus der Gruppe der schwefelhaltigen organischen Verbindungen, insbesondere Natriumthiosalicylsäure, Thiosorbit, Cystein, N-Acetyl-L-cystein, Glutathion, Cyteinhydrochlorid, Cysteamin, Cystin, Methionin, Glutathion, S-Acetylglutathion, Thioglycerol, Thioharnstoff, Thiolactat; und/oder EDTA, EGTA sowie Kombinationen hieraus, bevorzugt in einer Menge von 0,0001 - 5 Gew.-%, vorzugsweise 0,0001-1 Gew.-%, bevorzugt 0,001-0,5 Gew.-%, bezogen auf die gesamte Zusammensetzung.

15. Ophthalmologische Zusammensetzung nach einem der vorhergehenden Ansprüche, **dadurch gekennzeichnet, dass** sie
a) phosphatfrei und/oder frei von Calciumionen ist, und/oder
b) einen Chelatbildner für Calciumionen, bevorzugt aus der Gruppe der Citratsalze, Citronensäure, EDTA, EGTA und Mischungen davon enthält, bevorzugt in einer Konzentration von 0,05-10 Gew-%, bevorzugt 0,1 Gew-%, besonders bevorzugt 0,5-3 Gew-% bezogen auf das Gesamtgewicht der pharmazeutischen Zusammensetzung.

16. Ophthalmologische Zusammensetzung nach einem der vorhergehenden Ansprüche, enthaltend mindestens einen weiteren Bestandteil, ausgewählt aus der Gruppe bestehend aus
a) pharmakologisch geeigneten Trägerstoffen, insbesondere Wasser, Fettsäureester, wie Isopropylpalmitat und/oder Isopropylmyristat; Wachse; Fette; Vaseline; Paraffine; Mineralöl und/oder Pflanzenöl, bevorzugt in einer Menge zwischen 50 und 95 Gew.-%, bevorzugt zwischen 75 und 90 Gew.-%, bezogen auf die gesamte Zusammensetzung,
b) mindestens einen pflanzlichen anti-inflammatorischen und/oder antioxidativen Stoff, ausgewählt aus der Gruppe bestehend aus Flavonoiden (z.B. Rutin, Quercetin, Curcurmin), Isoflavonoiden (z.B. Silymarin), Polyphenole (z.B. Resveratol), Anthocyanen, Triterpenen, Monoterpenalkoholen, Phenolcarbonsäuren, Carotinoiden (z.B. β-Carotin, α-Carotin, Lycopin, β-Cryptoxanthin, Lutein, Zeaxanthin), Retinoide (z.B. Tretinoin), Tocopherolen (Vitamin E) und Biotin, Vitamine A, C, D, K, Coenzym Q (=Q10) Carnitin, N-Acetyl-Carnitin, Carnesol, Ubichinon und/oder Taurin, bevorzugt in einer Menge zwischen 0,01 und 5 Gew.-%, bezogen auf die gesamte Zusammensetzung,
c) mindestens einen pflanzliche Stoff mit antioxidativer, anti-inflammatorischer und/oder antiallergischer Wirkung, z.B. pflanzliche Einzelstoffe, Stoffgemische, ein flüssiger oder fester Extrakt, ein Destillat oder ein Öl oder ätherisches Öl, bevorzugt aus Pflanzen der Gattungen oder Arten Rosmarin, Sanddorn, Myrrhe, Eupharis (Augentrost), Kamille, Arnika, Ringelblume, Thymian, Echina, Calendula, Teebaum, Teestrauch, Apfelbeere (Aronia), Ginkgo, Ginseng, Heidelbeere, Holunder, Lavendel, Anis,
d) mindestens einen Wirkstoff, ausgewählt aus der Gruppe bestehend aus Antibiotika, abschwellenden Medikamenten, nichtsteroidalen Antiphlogistika, Virustatica, Antiseptika, Kortison, antiallergischen Wirkstoffen, Prostaglandin-Analoga, Wirkstoffen aus der Wirkstoffklasse der Antihistaminika und/oder der Corticosteroide, antiallergischen Wirkstoffe, Pantothensäurederivaten, nichtsteroidalen Entzündungshemmer, Vaskokonstriktoren und/oder Anti-Glaucom-Wirkstoffen in einer pharmakologisch Wirksamen Konzentration,
e) mindestens einen Gelbildner, ausgewählt aus der Gruppe bestehend aus natürlichen oder synthetischen Polymeren, bevorzugt in einer Menge zwischen 0,01 und 5 Gew.-%, bezogen auf die gesamte Zusammensetzung.
f) mindestens ein Verdickungsmittel, bevorzugt in einer Menge zwischen 0,5 und 5 Gew.-%, bezogen auf die gesamte Zusammensetzung.
g) mindestens ein Feuchthaltemittel,
h) mindestens eine Hilfsstoff, ausgewählt aus der Gruppe bestehend aus anorganischen Puffersubstanzen, organischen Puffersubstanzen, anorganischen Salzen, organischen Salzen, Viskositätsreglern, Lösungsmitteln, Lösungsvermittlern, Lösungsbeschleunigern, Salzbildnern, Viskositäts- und Konsistenzbeeinflussern, Solubilisatoren, Benetzern, Spreizmitteln, Füll- und Trägerstoffen, Osmolaritätsreglern sowie Mischungen davon, sowie
i) Kombinationen aus den zuvor genannten Bestandteilen.

17. Ophthalmologische Zusammensetzung nach einem der vorhergehenden Ansprüche zur topischen Applikation am Auge, insbesondere durch Einträufeln oder Eintropfen ins Auge bzw. auf die Augenoberfläche, Einsprühen oder Aufsprühen ins Auge bzw. auf die Augenoberfläche und/oder Einbringen auf die Innenseite des unteren Augenlids bzw. in den Bindehautsack, wobei die Applikation bevorzugt 1 mal täglich bis 4 mal stündlich, bevorzugt 2 mal täglich bis 12 mal täglich erfolgt.

18. Ophthalmologische Zusammensetzung nach einem der vorhergehenden Ansprüche, **dadurch gekennzeichnet, dass** die chirurgischen Eingriffe ausgewählt sind aus der Gruppe bestehend aus chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen, und Eingriffen an der Bindehaut.

19. Fluidspender für ein keimfreies Fluid, mit
a) einem Durchgang, der eine Einlassöffnung für ein in einem Vorratsbehälter aus einem flexiblen Material enthaltenes Fluid und eine Ausströmöffnung zum Ausgeben des Fluids verbindet und darin wenigstens eine oligodynamisch aktive Substanz besitzt, die mit dem Fluid in Kontakt ist;
b) einer Dosierpumpe, die ohne Luftdruckkompensation arbeitet, umfassend ein Einlassventil zum Schließen der Einlassöffnung, wobei das Einlassventil ein Material aufweist, das mit dem Fluid über eine oligodynamisch aktive Substanz wechselwirken kann; und
c) eine Federeinrichtung, die in Kontakt mit dem Fluid steht, wobei das Einlassventil und die Federeinrichtung ein rostfreies Stahlmaterial als eine oligodynamisch aktive Substanz aufweisen und eine Dekontaminierungseinrichtung im oberen Teil des Auslasskanals vorgesehen ist, wobei die Dekontaminierungseinrichtung ein Material aufweist, das mit dem Fluid über eine oligodynamische Substanz wechselwirken kann, die ausgewählt ist aus der Gruppe bestehend aus Silber, Silbersalzen, anderen Silberverbindungen, Legierungen und Nanomeren davon in entweder metallischer oder Salzform oder als eine chemische Verbindung daraus,
**dadurch gekennzeichnet, dass**
das im Vorratsbehälter enthaltene Fluid eine ophthalmologische Zusammensetzung nach einem der Ansprüche 1 bis 18 ist.

20. Verwendung einer Zusammensetzung, enthaltend
a) mindestens eine ω-3-Fettsäure und/oder ein ω-3-Fettsäurederivat ausgewählt aus der Gruppe bestehend aus Lipiden, Oxygenierungsprodukten, Carboxylatsalzen, Estern, Mono-, Di- oder Triglyceriden, Amiden, sonstigen pharmakologisch akzeptablen Carbonsäurederivaten und Mischungen hiervon, sowie
b) mindestens ein Glucosaminoglucan, hiervon abgeleitete pharmakologisch akzeptable Derivate, sowie Mischungen hiervon,
als Kosmetikum, insbesondere als Gesichtskosmetikum.
